# EUROPEAN PATENT SPECIFICATION

(11) **EP 3 364 970 B1**
(45) Date of publication and mention of the grant of the patent: **07.02.2024**
(21) Application number: 16858441.5
(22) Date of filing: 24.10.2016
(51) Int. Cl.: A61K 31/436, A61K 38/13, A61K 38/46, A61K 48/00, A61P 37/06

(54) **GENE THERAPY FOR USE IN TREATING LYSOSOMAL STORAGE DISEASE**
GENTHERAPIE ZUR VERWENDUNG BEI DER BEHANDLUNG VON LYSOSOMALE SPEICHERKRANKHEIT
THÉRAPIE GÉNIQUE POUR L'UTILISATION DANS LE TRAITEMENT DE LA MALADIE LYSOSOMALE

(30) Priority: 23.10.2015 US 201562245824 P
(43) Date of publication of application: 29.08.2018
(73) Proprietor: University of Iowa Research Foundation, Iowa City, IA 52242 (US)
(72) Inventor: DAVIDSON, Beverly, L., Iowa City, Iowa 52242 (US); CHEN, Yong Hong, Iowa City, Iowa 52242 (US); TECEDOR, Luis, Iowa City, Iowa 52242 (US)
(74) Representative: PATERIS Patentanwälte PartmbB
(86) International application number: PCT/US2016/058484
(87) International publication number: WO 2017/070678

(56) References cited:
- WO-A1-2014/186579
- WO-A2-2015/013148
- US-A1- 2014 088 179
- PASSINI MARCO A ET AL: "Intracranial delivery of CLN2 reduces brain pathology in a mouse model of classical late infantile neuronal ceroid lipofuscinosis", JOURNAL OF NEUROSCI, THE SOCIETY, WASHINGTON, DC, US, vol. 26, no. 5, 1 February 2006 (2006-02-01), pages 1334-1342, XP009184135, ISSN: 1529-2401, DOI: 10.1523/JNEUROSCI.2676-05.2006
- G WATSON ET AL: "Intrathecal administration of AAV vectors for the treatment of lysosomal storage in the brains of MPS I mice", GENE THERAPY, vol. 13, no. 11, 16 February 2006 (2006-02-16), pages 917-925, XP055540175, GB ISSN: 0969-7128, DOI: 10.1038/sj.gt.3302735
- JIANG ET AL.: 'Effects of transient immunosuppression on adenoassociated, virus-mediated, liver-directed gene transfer in rhesus macaques and implications for human gene therapy' BLOOD vol. 108, no. 10, 25 July 2006, pages 3321 - 3328, XP055175201
- SHIN ET AL.: 'A simplified immune suppression scheme leads to persistent micro-dystrophin expression in Duchenne muscular dystrophy dogs' HUM GENE THER vol. 23, no. 2, 14 December 2011, pages 202 - 209, XP055323688
- WANG ET AL.: 'Sustained AAV-mediated dystrophin expression in a canine model of Duchenne muscular dystrophy with a brief course of immunosuppression' MOLECULAR THERAPY vol. 15, no. 6, 10 April 2007, pages 1160 - 1166, XP055152230

## Description

### INTRODUCTION

Gene transfer is now widely recognized as a powerful tool for analysis of biological events and disease processes at both the cellular and molecular level. More recently, the application of gene therapy for the treatment of human diseases, either inherited (e.g., ADA deficiency) or acquired (e.g., cancer or infectious disease), has received considerable attention.

Traditionally, gene therapy has been defined as a procedure in which a therapeutic gene is introduced into cells of a mammal in order to correct an inborn genetic error. Although more than 4500 human diseases are currently classified as genetic, specific mutations in the human genome have been identified for relatively few of these diseases. Until recently, these rare genetic diseases represented the exclusive targets of gene therapy efforts. Accordingly, most of the NIH approved gene therapy protocols to date have been directed toward the introduction of a functional copy of a defective gene into the somatic cells of an individual having a known inborn genetic error. Only recently, have researchers and clinicians begun to appreciate that most human cancers, certain forms of cardiovascular disease, and many degenerative diseases also have important genetic components, and for the purposes of designing novel gene therapies, should be considered "genetic disorders." Therefore, gene therapy has more recently been broadly defined as the correction of a disease phenotype through the introduction of new genetic information into the affected organism.

In *in vivo* gene therapy, a transferred gene is introduced into cells of the recipient organism *in situ* that is, within the recipient. *In vivo* gene therapy has been examined in several animal models. Several recent publications have reported the feasibility of direct gene transfer *in situ* into organs and tissues such as muscle, hematopoietic stem cells, the arterial wall, the nervous system, and lung. Direct injection of DNA into skeletal muscle, heart muscle and injection of DNA-lipid complexes into the vasculature also has been reported to yield a detectable expression level of the inserted gene product(s) *in vivo.*

Treatment of diseases of the central nervous system, e.g., inherited genetic diseases of the brain, remains an intractable problem. Examples of such are the lysosomal storage diseases and Alzheimer's disease. Collectively, the incidence of lysosomal storage diseases (LSD) is 1 in 10,000 births worldwide, and in 65% of cases, there is significant central nervous system (CNS) involvement. Proteins deficient in these disorders, when delivered intravenously, do not cross the blood-brain barrier, or, when delivered directly to the brain, are not widely distributed. Thus, therapies for the CNS deficits need to be developed.

WO 2015/013148 A2 describes the use of AAV particles for delivering a nucleic acid encoding the lysosomal hydrolase TPP1 to the brain of a mammal in order to treat the lysosomal storage disease LINCL. It also describes that the AAV particles can be administered in combination with a single immunosuppressive agent, namely mycophenolate, and that the use of the immunosuppressive agent extends the duration of TPP1 enzyme activity in the CSF. Jiang et al. "Effects of transient immunosuppression on adenoassociated, virus-mediated, liver-directed gene transfer in rhesus macaques and implications for human gene therapy", Blood, vol. 108, no. 10, 2006, pages 3321-3328, describes peripheral administration of AAV particles and that AAV particle therapy can be combined with administration of two immunosuppressive agents. It is disclosed that there was either no net effect or an increase in neutralizing antibody titer/response when AAV and immunosuppressive agents were administered to subjects. WO 2014/186579 A1 describes that AAV particle therapy can be combined with administration of a single immunosuppressive agent, namely cyclophosphamide. It is disclosed that the use of the immunosuppressive agent treatment leads to an increased activity of the therapeutic protein. US 2014/088179 A1 describes an AAV vector, comprising an AAV ITR sequence, and a nucleic acid encoding TPP1. Passini et al. "Intracranial delivery of CLN2 reduces brain pathology in a mouse model of classical late infantile neuronal ceroid lipofuscinosis", Journal of Neuroscience., vol. 26, no. 5, 2006, pages 1334-1342, also describes an AAV vector, comprising an AAV ITR sequence, and a nucleic acid encoding TPP1.

### SUMMARY

The present invention is defined by the claims. The references to the methods of treatment by therapy or surgery of this description are to be interpreted as references to compounds, pharmaceutical compositions and medicaments of the present invention for use in those methods.

The present invention relates to a plurality of AAV particles, said AAV particles (i) comprising an AAV capsid protein and a nucleic acid inserted between a pair of AAV inverted terminal repeats (ITRs), said nucleic acid encoding a polypeptide, wherein said polypeptide comprises a tripeptidylpeptidase 1 (TPP1) polypeptide having at least 90% identity to an amino acid sequence set forth in SEQ ID NO:5 or a pro-enzyme thereof, and (ii) being capable of transducing cells of said mammal and providing expression of said polypeptide; and a first immunosuppressive agent and a second immunosuppressive agent, for use in treating a lysosomal storage disease (LSD) in a mammal, wherein the plurality of AAV particles is to be administered to the brain or spine of said mammal, wherein said first immunosuppressive agent and said second immunosuppressive agent are to be administered to said mammal, wherein said first immunosuppressive agent is cyclosporine and said second immunosuppressive agent is mycophenolate mofetil (MMF), wherein at least one of said first immunosuppressive agent and said second immunosuppressive agent are to be administered to said mammal prior to administration of said AAV particles, and wherein neutralizing antibody titer in said mammal is reduced by administration of said first immunosuppressive agent and said second immunosuppressive agent.

Described herein are methods of treating a mammal having a lysosomal storage disease (LSD). In one embodiment a method described herein comprises: (a) administering to the brain or spine of a mammal a plurality of AAV particles,
said AAV particles (i) comprising an AAV capsid protein and a nucleic acid inserted between a pair of AAV inverted terminal repeats (ITRs), said nucleic acid encoding a polypeptide having lysosomal hydrolase activity, and (ii) being capable of transducing cells of said mammal and providing expression of said polypeptide; and
(b) administering to said mammal a first immunosuppressive agent and a second immunosuppressive agent. In a particular aspect, the first immunosuppressive agent and the second immunosuppressive agent are administered to the mammal prior to administration of the AAV particles.

In particular embodiments, the polypeptide has tripeptidyl-peptidase 1 (TPP1) activity. In particular aspects, the polypeptide comprises TPP1, a pro-enzyme thereof, or an enzymatically active variant thereof.

In further particular embodiments, the first immunosuppressive agent is administered prior to administration of the AAV particles and the second immunosuppressive agent is administered prior to, concurrently with, or after administration of said AAV particles.

In particular aspects, the first immunosuppressive agent is administered at least about two weeks prior to administration of said AAV particles and the second immunosuppressive agent is administered about two weeks before or within 40 days after administration of the AAV particles.

In particular aspects, the first immunosuppressive agent comprises cyclosporine. In particular aspects, the second immunosuppressive agent comprises mycophenolate or a derivative thereof (e.g., mycophenolate mofitil (MMF)). According to the claimed invention, the first immunosuppressive agent is cyclosporine and the second immunosuppressive agent is MMF.

In certain embodiments, the first immunosuppressive agent (e.g., cyclosporine) is administered at a dosage of about 5-20 mg/kg twice a day for a period of at least 3 months.

In certain embodiments, the dose of the first immunosuppressive agent (e.g., cyclosporine) is reduced after a 1-2 months after administration of the AAV particles.

In certain embodiments, the second immunosuppressive agent (e.g., mycophenolate or a derivative thereof) is administered at a dosage of about 5-20 mg/kg a day.

In additional particular embodiments, tripeptidyl-peptidase 1 (TPP1) activity in the cerebrospinal fluid of the mammal is detectable at a level of at least 5 pmol TPP1/mg protein for greater than 350 days.

In additional embodiments, cells (e.g., ependymal cells) comprising the cerebrospinal fluid (CSF) of the mammal are transduced by said AAV particles. In particular aspects, cells (e.g., ependymal cells) transduced with the AAV particles express and secrete the polypeptide into the CSF of said mammal.

In further embodiments, administration of the AAV particles comprises administration to the cisterna magna, intraventricular space, brain ventricle, subarachnoid space, intrathecal space and/or ependyma of said mammal.

In still further embodiments, administration of the AAV particles comprises administration to the cerebral spinal fluid (CSF) of said mammal.

In still additional embodiments, administration of the AAV particles comprises contacting ependymal cells of said mammal with the AAV particles.

In certain aspects, administration of the AAV particles comprises contacting a pial cell, endothelial cell, or meningeal cell of said mammal with said AAV particles.

In certain embodiments, administration of the AAV particles comprises injection of the AAV particles into a tissue or fluid of the brain or spinal cord of said mammal.

In certain embodiments, administration of the AAV particles comprises injection of the AAV particles into cerebral spinal fluid of said mammal.

In certain embodiments, the mammal is a non-rodent mammal. In particulars aspects, the non-rodent mammal is a primate, horse, sheep, goat, pig, or dog. In particulars aspects, the non-rodent mammal is a human (e.g., a child, for example, from about 1 to about 4 years of age).

In certain embodiments, the LSD is infantile or late infantile ceroid lipofuscinoses (LINCL), neuronopathic Gaucher, Juvenile Batten, Fabry, MLD, Sanfilippo A, Hunter, Krabbe, Morquio, Pompe, Niemann-Pick C, Tay-Sachs, Hurler (MPS-I H), Sanfilippo B, Maroteaux-Lamy, Niemann-Pick A, Cystinosis, Hurler-Scheie (MPS-I H/S), Sly Syndrome (MPS VII), Scheie (MPS-I S), Infantile Batten, GM1 Gangliosidosis, Mucolipidosis type II/III, or Sandhoff disease.

In particular embodiments, a symptom associated with the LSD is delayed, reduced, decreased or ameliorated. In certain aspects, a symptom is one or more of proionceptive response, nystagmus, menace, pupillary light reflex, cerebellar ataxia and intention tremor.

In particular embodiments, onset of a symptom associated with the LSD is delayed by 5-10, 10-25, 25-50 or 50-100 days, e.g., when the first immunosuppressive agent and second immunosuppressive agent are administered, compared to administration of the second immunosuppressive agent without prior administration of the first immunosuppressive agent.

In particular embodiments, onset of a loss of cognitive function associated with the LSD is delayed by 5-10, 10-25, 25-50 or 50-100 days when the first immunosuppressive agent and second immunosuppressive agent are administered, compared to administration of the second immunosuppressive agent without prior administration of the first immunosuppressive agent.

In certain embodiments, lifespan of a mammal having the LSD is extended, for example, by 5-10, 10-25, 25-50 or 50-100 days when the first immunosuppressive agent and second immunosuppressive agent are administered, compared to administration of the second immunosuppressive agent without prior administration of the first immunosuppressive agent.

In certain embodiments, the AAV particles are administered at a dose of about 1×108 to about 1×1015 vg/kg.

In certain embodiments, neutralizing antibodies (e.g., against AAV vector particles or polypeptide having lysosomal hydrolase activity) are not detected in CSF of the mammal for at least 30, 60, 90, 120 or more days after administration of the AAV particles. In particular aspects, neutralizing antibodies (e.g., against AAV vector particles or polypeptide having lysosomal hydrolase activity) are not detected in CSF of said mammal for at least 250 days after said administration of said AAV particles.

In certain embodiments, the polypeptide is expressed in the spleen or heart of said mammal.

In certain embodiments, the polypeptide is expressed in the cerebellum of said mammal.

In certain embodiments, the AAV particles are AAV1, AAV2, AAV3, AAV4, AAV5, AAV6, AAV7, AAV8, AAV9, AAV10, AAV11, AAV12, AAV-rh74, AAV-rh10 and AAV-2i8 particles.

In certain embodiments, one or more of the AAV ITRs are an AAV1, AAV2, AAV3, AAV4, AAV5, AAV6, AAV7, AAV8, AAV9, AAV10, AAV11, AAV12, AAV-rh74, AAV-rh10 and AAV-2i8 ITR.

Further described are AAV vectors and AAV vector particles. In one embodiment, an AAV vector includes an AAV ITR sequence, a nucleic acid encoding a polypeptide having lysosomal hydrolase activity (e.g., TPP1), and an expression control element driving expression of the nucleic acid encoding the polypeptide having lysosomal hydrolase activity (e.g., TPP1).

In one embodiment, an AAV particle includes the AAV vectors as set forth herein.

In certain embodiments, an AAV vector or AAV particle includes an AAV ITR sequence such as one or more AAV2 ITRs.

In certain embodiments, an AAV vector or AAV has a nucleic acid encoding mammalian TPP1. In certain aspects, the nucleic acid encodes human TPP1. In certain aspects, the nucleic acid encodes a protein having 80% or more identity to human TPP1 set forth as SEQ ID NO:5. In certain aspects, the nucleic acid encodes human TPP1 set forth as SEQ ID NO:5. According to the claimed invention, the nucleic acid encodes a polypeptide, wherein said polypeptide comprises a TPP1 polypeptide having at least 90% identity to an amino acid sequence set forth in SEQ ID NO:5 or a pro-enzyme thereof.

In certain embodiments, an expression control element comprises a CMV enhancer.

In certain embodiments, an expression control element comprises a beta actin promoter. In particular aspects, an expression control element comprises a chicken beta actin promoter.

In certain embodiments, an expression control element comprises a CMV enhancer and a chicken beta actin promoter.

In certain embodiments, an expression control element comprises a sequence having 80% or more identity to SEQ ID NO:9. In particular aspects, an expression control element comprises SEQ ID NO:9.

In certain embodiments, an AAV particle has a capsid sequence comprising a VP1, VP2 and/or VP3 capsid sequence having 90% or more identity to AAV1, AAV2, AAV3, AAV4, AAV5, AAV6, AAV7, AAV8, AAV9, AAV10, AAV11, Rh10, Rh74 or AAV-2i8 VP1, VP2 and/or VP3 sequences.

In certain embodiments, an AAV particle has a VP1 capsid sequence having 80% or more identity to AAV2 VP1 capsid sequence, or having 80% or more identity to AAV2, wherein the capsid sequence has a tyrosine at positions 444, 500 and/or 730 substituted with an amino acid that is not tyrosine. In particular aspects, the capsid sequence has 80% or more identity to AAV2 VP1 capsid sequence, wherein the capsid sequence has a tyrosine at positions 444, 500 and/or 730 substituted with phenylalanine. In more particular aspects, the capsid sequence comprises an AAV2 VP1 capsid sequence in which a tyrosine at positions 444, 500 and/or 730 has been substituted with a phenylalanine.

In certain embodiments, an AAV particle has a, VP2 or VP3 capsid sequence selected from any of: AAV1, AAV2, AAV3, AAV4, AAV5, AAV6, AAV7, AAV8, AAV9, AAV10, AAV11, Rh10, Rh74 or AAV-2i8 AAV serotypes.

### DESCRIPTION OF THE DRAWINGS

FIGS.1A-C together show an alignment of VP1 of AAV2 (SEQ ID NO:1; AVV2capPro) and VP1 of AAV4 (SEQ ID NO:2; AVV4capPro).

FIGS.2A-G together show an alignment of AAV2 (SEQ ID NO:3; AVV2capNuc) and AAV4 (SEQ ID NO:4; AVV4capNuc1) nucleotides (i.e., the ORF encoding VP1, VP2 and VP3) based on the sequences from AAV2 (NCBI Accession No. NC_001401) and AAV4 (NCBI Accession NO. NC_001829).

FIG.3 shows the Human TPP1 amino acid sequence (SEQ ID NO:5).

FIGS.4A-C together show the Human TPP1 nucleic acid sequence (SEQ ID NO:6).

FIG.5 shows the *Macaca mulatta* TPP1 amino acid sequence (SEQ ID NO:7).

FIG.6 shows the *Macaca fascicularis* TPP1 amino acid sequence (SEQ ID NO:8).

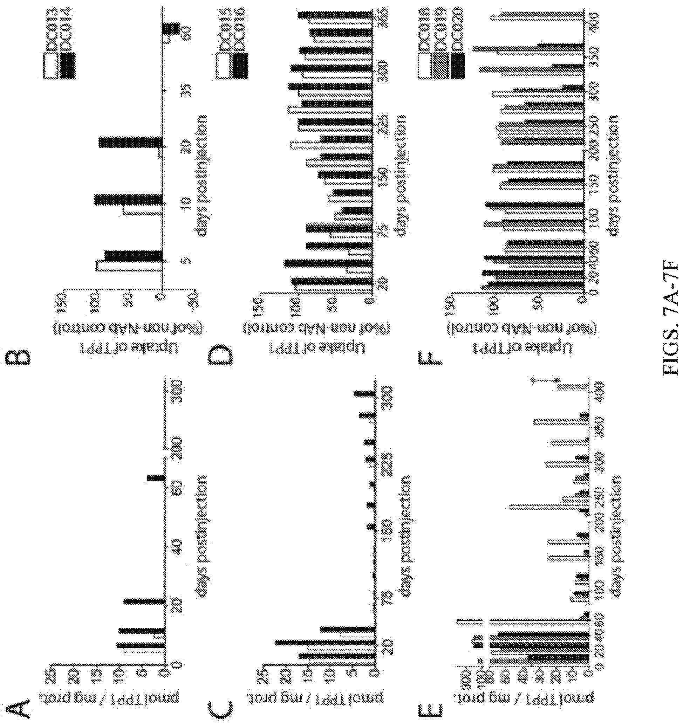

FIGS.7A-7F show TPP1 and TPP1 neutralizing antibodies (NAbs) levels in CSF. Elevated TPP1 levels were measured by activity assay (FIGS.7A, 7C and 7E), and NAbs levels were measured by exposure of TPP1-deficient mouse embryonic fibroblasts to a mixture of purified TPP1 plus dog CSF collected before or after treatment, relative to untreated dog CSF (FIGS.7B, 7D, and 7F). TPP1 activity and NAb levels were measured in dogs that were given immunosuppression treatment: at day 44 relative to vector infusion (FIGS.7A and 7B); at day 33 relative to vector infusion (FIGS.7C and 7D) and at day -5 relative to vector infusion (FIGS.7E and 7F; DC019 shows 0.3 pmol/mg protein, which is 100% of normal).

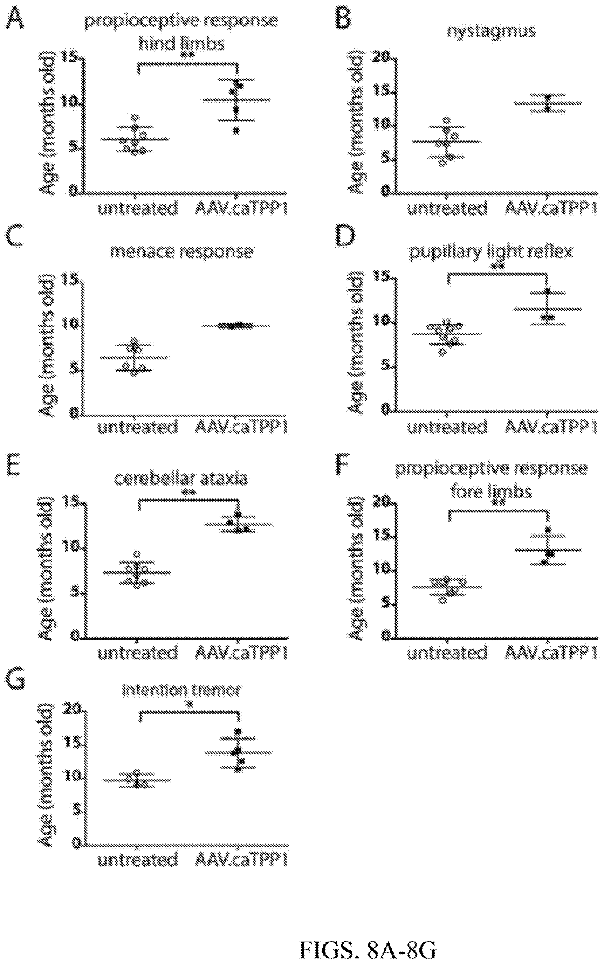

FIGS.8A-8G show the effect of AAV2.caTPP1 treatment on onset of clinical symptoms in TPP1-deficient dogs: (FIG.8A) onset of impaired proprioceptive responses in the pelvic limbs was delayed by 4.5 months on average in treated dogs compared to untreated dogs; (FIG.8B) pathological nystagmus did not appear in 3 of 5 treated dogs; (FIG.8C) onset of menace response deficits were delayed in treated dogs compared to untreated dogs; (FIG.8D) pupillary light reflex abnormalities appeared 3.1 months later on average in treated dogs compared to untreated dogs; (FIG.8E) cerebellar ataxia was detected 5.4 months later in treated dogs compared to untreated dogs; (FIG.8F) onset of impaired proprioceptive responses in the fore limbs was delayed in treated dogs compared to untreated dogs; (FIG.8G) treated dogs start showing intention tremors 4.1 months later than untreated dogs. *p<0.05, **p<0.01 using nonparametric Mann-Whitney test.

FIG.9 shows the effect of AAV2.caTPP1 treatment on onset of cognitive deficits. AAV.caTPP1 treated dogs outperformed untreated dogs from 4 months (1 month post-injection) onward.

FIGS.10A-10B show TPP1 activity levels in brain parenchyma at the experimental endpoints: (FIG.10A) TPP1 activity levels in ependymal; (FIG. 10B) TPP1 activity levels in periventricular or meningeal regions. TPP1 levels are relative to that found in normal dogs.

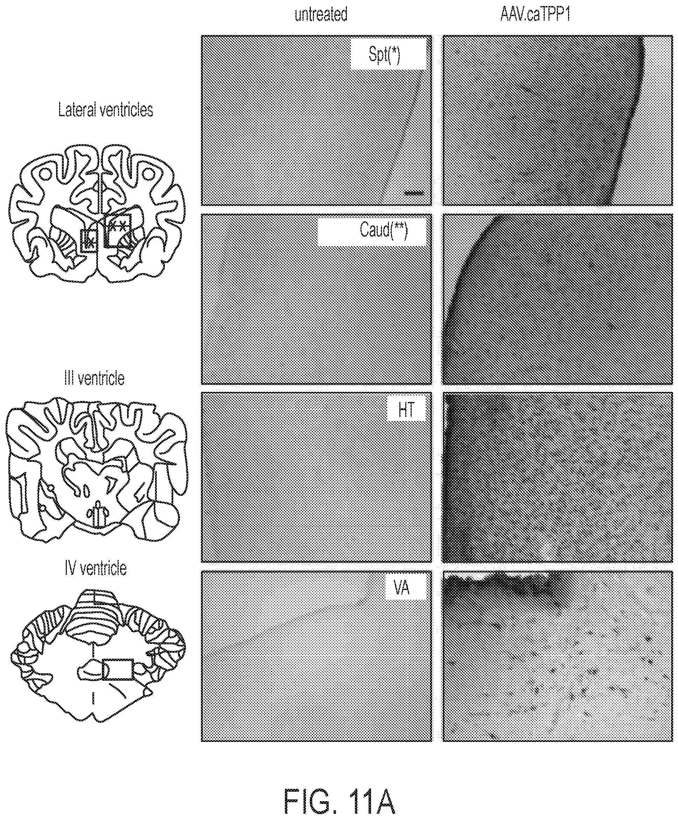

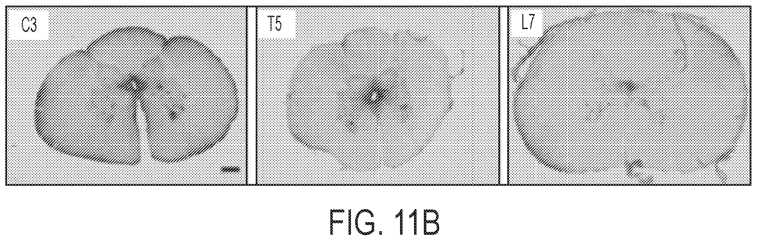

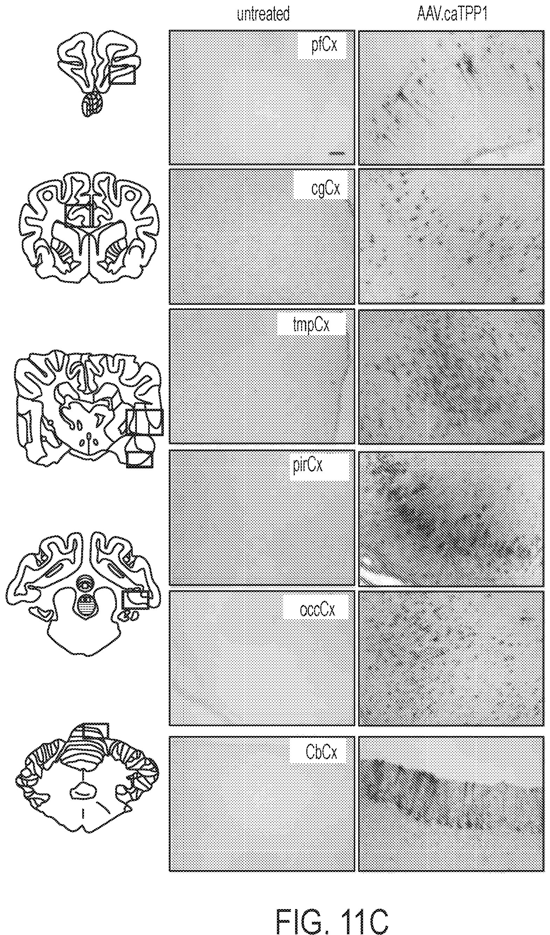

FIGS.11A-11C show biodistribution of caTPP1 in AA V.caTPP1 infused dogs: (FIG.11A) immunohistochemical staining for canine TPP1 in treated (Right) and untreated (Middle) TPP1-deficient dogs. TPP1-immunopositive cells in the septum (Spt) and caudate nucleus (Caud) in the forebrain, hypothalamus (HT) at the level of the 3^{rd} ventricle, and vestibular area (VA) near the 4th ventricle are shown. Scale bar = 100 µm. Cross-section areas for I. lateral ventricles (first and second row panels), III. ventricle (third row panels) and IV ventricle (fourth row panels) are indicated; (FIG.11B) TPP1-immunopositive cells near the central canal and neurons along the spinal cord; cervical 3 (C3), thoracic 5 (T5), and lumbar 7 (L7). Scale bar = 400 µm; (FIG. 11C) immunohistochemical staining for canine TPP1 in treated (Right) and untreated (Middle) TPP1-deficient dogs. Cross-section areas are indicated to the left of the panels. TPP1-immunopositive cells TPP1- immunopositive cells from the caudal to rostral cortical regions are shown. Scale bar = 100 µm.

FIG. 12 shows the effect of AAV.caTPP1 treatment on astrocytic activation. Glial astrocytosis measured by immunoreactivity for glial fibrillary acidic protein (GFAP) was diminished in treated dogs compared to untreated TPP1-deficient dogs. mCx = motor cortex; cgCx = cingulate cortex. Scale bar = 1 mm.

FIGS. 13A-13B show: (FIG. 13A) decreased levels of autofluorescent storage and immunolabeling for ATP synthase subunit C accumulation in AAV2.caTPP1 treated dogs compared to untreated TPP1-deficient dogs. Scale bar = 25 µm and 100 µm, respectively; (FIG.13B) p62 and SCMAS as quantified by Western blot in thalamus (Th), cerebellum (CB), and occipital cortex (occCx). Results of normal dogs (black bars) and AAV.caTPP1 treated dogs (open bars) were normalized to p62 and SCMAS levels in untreated dogs. AAV.caTPP1 were grouped by day of MMF treatment relative to vector infusion.

FIG.14 shows TPP1 levels in peripheral organs. TPP1 levels were measured by TPP1 activity assay in homogenized samples of the indicated peripheral tissues of untreated (-), AA V.caTPP1-treated (+), and normal dogs (+/+). Increased TPP1 activity was detected in spleen and heart samples of AA V.caTPP1-treated compared to untreated TPP1-deficient dogs. *p<0.05 using nonparametric Kruskal-Wallis test.

FIG.15 shows TPP1 levels in CSF of normal dogs. TPP1 levels were measured by TPP1 activity levels in normal canine CSF.

FIG.16 shows the effect of AA V.caTPP 1 treatment on life span. Survival plot of untreated TPP1-null dogs (black line) and AA V.caTPP1 injected animals treated with MMF treatment at 33 days post-injection (dashed line) or 5 days pre-injection (dotted line).

### DETAILED DESCRIPTION

Described herein are methods of treating a lysosomal storage disease (LSD) in a mammal. In certain embodiments a mammal in need of a treatment described herein is a mammal that has, or is suspected of having a LSD. In certain embodiments a method or composition described herein is used to treat, prevent or delay the onset of one or more symptoms of an LSD.

Non-limiting examples of LSDs include Infantile Lipofuscinosis or Late infantile Neuronal Ceroid Lipofuscinosis (LINCL), Gaucher, Juvenile Batten, Fabry, MLD, Sanfilippo A, Late Infantile Batten, Hunter, Krabbe, Morquio, Pompe, Niemann-Pick C, Tay-Sachs, Hurler (MPS-I H), Sanfilippo B, Maroteaux-Lamy, Niemann-Pick A, Cystinosis, Hurler-Scheie (MPS-I H/S), Sly Syndrome (MPS VII), Scheie (MPS-I S), Infantile Batten, GM1 Gangliosidosis, Mucolipidosis type II/III, or Sandhoff disease.

LSDs are often caused by a genetic abnormality (e.g., mutation, deletion, insertion) in the gene encoding a tripeptidyl peptidase-1 (TPP1) enzyme thereby leading to a deficiency of TPP1 enzyme activity. In humans, TPP1 is encoded by the CLN2 gene, sometimes called the TPP1 gene. For example, Late infantile Neuronal Ceroid Lipofuscinosis (LINCL) is a childhood neurodegenerative disease caused most often by deficiency of TPP1 activity, due to mutations in CLN2. Development is normal up to ages 2-4 years after which manifestations present as motor and mental decline, seizure disorder and visual deficits. Death generally occurs within the first decade of life. Most cases of LINCL are due to mutations in CLN2, which induce a deficiency of the soluble lysosomal enzyme tripeptidyl peptidase-1 (TPP1). TPP1 is synthetized as a mannose-6-phophate pro-enzyme and, similar to other soluble lysosomal hydrolases, the pro-enzyme is largely targeted to the lysosome but can also be released from the cell via the secretory pathway. As such, cellular uptake by the same or neighboring cells, and subsequent lysosomal delivery and activation of the proenzyme to the active form, can occur.

Described herein are methods of treating a mammal having, or suspected of having an LSD by administering, directly to a tissue or fluid of the central nervous system, AAV particles that direct the expression of polypeptide having TPP1 activity (referred to herein as AAV-TPP1 particles). In certain embodiments, AAV-TPP1 particles are administered in conjunction with one or more immunosuppressive agents. Disclosed herein are data showing efficacy of direct AAV delivery to the brain and/or spinal cord in an animal model of a lysosomal storage disorder.

Any suitable mammal can be treated by a method or composition described herein. Non-limiting examples of mammals include humans, non-human primates (e.g., apes, gibbons, chimpanzees, orangutans, monkeys, macaques, and the like), domestic animals (e.g., dogs and cats), farm animals (e.g., horses, cows, goats, sheep, pigs) and experimental animals (e.g., mouse, rat, rabbit, guinea pig). In certain embodiments a mammal is a human. In certain embodiments a mammal is a non-rodent mammal (e.g., human, pig, goat, sheep, horse, dog, or the like). In certain embodiments a non-rodent mammal is a human. A mammal can be any age or at any stage of development (e.g., an adult, teen, child, infant, or a mammal in utero). A mammal can be male or female. In certain embodiments a mammal can be an animal disease model, for example, animal models used for the study of LSDs.

Subjects treated by a method or composition described herein include adults (18 years or older) and children (less than 18 years of age). Children also include infants. Children range in age from less than 2 years old, or from 2-4, 4-6, 6-18, 8-10, 10-12, 12-15 and 15-18 years old. Infants typically range from 1-12 months of age.

Adeno associated virus (AAV) is a small nonpathogenic virus of the parvoviridae family. AAV is distinct from the other members of this family by its dependence upon a helper virus for replication. AAV genomes been shown to stably integrate into host cellular genomes; possess a broad host range; transduce both dividing and non-dividing cells *in vitro* and *in vivo* and maintain high levels of expression of the transduced genes. AAV viral particles are heat stable, resistant to solvents, detergents, changes in pH, temperature, and can be concentrated on CsCl gradients or by other means. The AAV genome comprises a single-stranded deoxyribonucleic acid (ssDNA), either positive- or negative-sensed. In the absence of a helper virus, AAV may integrate in a locus specific manner, for example into the q arm of chromosome 19. The approximately 5 kb genome of AAV consists of one segment of single stranded DNA of either plus or minus polarity. The ends of the genome are short inverted terminal repeats which can fold into hairpin structures and serve as the origin of viral DNA replication.

To date, numerous serologically distinct AAVs have been identified, and more than a dozen have been isolated from humans or primates. The genome of most native AAVs often contain two open reading frames (ORFs), sometimes referred to as a left ORF and a right ORF. The left ORF often encodes the non-structural Rep proteins, Rep 40, Rep 52, Rep 68 and Rep 78, which are involved in regulation of replication and transcription in addition to the production of single-stranded progeny genomes. Two of the Rep proteins have been associated with the preferential integration of AAV genomes into a region of the q arm of human chromosome 19. Rep68/78 have been shown to possess NTP binding activity as well as DNA and RNA helicase activities. Some Rep proteins possess a nuclear localization signal as well as several potential phosphorylation sites. In certain embodiments the genome of an AAV (e.g., an rAAV) encodes some or all of the Rep proteins. In certain embodiments the genome of an AAV (e.g., an rAAV) does not encode the Rep proteins. In certain embodiments one or more of the Rep proteins can be delivered in trans and are therefore not included in an AAV particle comprising a nucleic acid encoding a polypeptide.

The ends of the AAV genome comprise short inverted terminal repeats (ITR) which have the potential to fold into T-shaped hairpin structures that serve as the origin of viral DNA replication. Accordingly, the genome of an AAV comprises one or more (e.g., a pair of) ITR sequences that flank its single stranded viral DNA genome. The ITR sequences often comprise about 145 bases each. Within the ITR region, two elements have been described which are thought to be central to the function of the ITR, a GAGC repeat motif and the terminal resolution site (trs). The repeat motif has been shown to bind Rep when the ITR is in either a linear or hairpin conformation. This binding is thought to position Rep68/78 for cleavage at the trs which occurs in a site- and strand-specific manner. In addition to their role in replication, these two elements appear to be central to viral integration. Contained within the chromosome 19 integration locus is a Rep binding site with an adjacent trs. These elements have been shown to be functional and necessary for locus specific integration. In certain embodiments an AAV (e.g., an rAAV) comprises two ITRs. In certain embodiments an AAV (e.g., an rAAV) comprises a pair of ITRs. In certain embodiments an AAV (e.g., an rAAV) comprises a pair of ITRs that flank (i.e., are at each 5' and 3' end) of a polynucleotide that at least encodes a polypeptide having TPP1 enzyme activity.

The AAV virion (particle) is a non-enveloped, icosahedral particle approximately 25 nm in diameter. The AAV particle comprises a capsid of icosahedral symmetry comprised of three related capsid proteins, VP1, VP2 and VP3, which interact together to form the capsid. The right ORF often encodes the capsid proteins VP1, VP2, and VP3. These proteins are often found in a ratio of 1:1:10 respectively, but may be in varied ratios, and are all derived from the right-hand ORF. The capsid proteins differ from each other by the use of alternative splicing and an unusual start codon. Deletion analysis has shown that removal or alteration of VP1 which is translated from an alternatively spliced message results in a reduced yield of infectious particles. Mutations within the VP3 coding region result in the failure to produce any single-stranded progeny DNA or infectious particles. An AAV particle is a viral particle comprising an AAV capsid. In certain embodiments the genome of an AAV particle encodes one, two or all VP1, VP2 and VP3 polypeptides.

The term "vector" refers to small carrier nucleic acid molecule, a plasmid, virus (e.g., AAV vector), or other vehicle that can be manipulated by insertion or incorporation of a nucleic acid. Such vectors can be used for genetic manipulation (i.e., "cloning vectors"), to introduce/transfer polynucleotides into cells, and to transcribe or translate the inserted polynucleotide in cells.

An "expression vector" is a specialized vector that contains a gene or nucleic acid sequence with the necessary regulatory regions needed for expression in a host cell. A vector nucleic acid sequence generally contains at least an origin of replication for propagation in a cell and optionally additional elements, such as a heterologous polynucleotide sequence, expression control element (e.g., a promoter, enhancer), intron, ITR(s), polyadenylation signal.

A viral vector is derived from or based upon one or more nucleic acid elements that comprise a viral genome. Particular viral vectors include adeno-associated virus (AAV) vectors. Also described are vectors comprising a nucleic acid sequence encoding a TPP1 polypeptide, variant or subsequence (e.g., a polypeptide fragment having TPP1 enzyme activity).

The term "recombinant," as a modifier of vector, such as recombinant viral, e.g., lenti- or parvo-virus (e.g., AAV) vectors, as well as a modifier of sequences such as recombinant polynucleotides and polypeptides, means that the compositions have been manipulated (i.e., engineered) in a fashion that generally does not occur in nature. A particular example of a recombinant vector, such as an AAV vector would be where a polynucleotide that is not normally present in the wild-type viral (e.g., AAV) genome is inserted within the viral genome. An example of a recombinant polynucleotide would be where a nucleic acid (e.g., gene) encoding a TPP1 polypeptide is cloned into a vector, with or without 5', 3' and/or intron regions that the gene is normally associated within the viral (e.g., AAV) genome. Although the term "recombinant" is not always used herein in reference to vectors, such as viral and AAV vectors, as well as sequences such as polynucleotides, recombinant forms including polynucleotides, are expressly included in spite of any such omission.

A recombinant viral "vector" or "AAV vector" is derived from the wild type genome of a virus, such as AAV by using molecular methods to remove the wild type genome from the virus (e.g., AAV), and replacing with a non-native nucleic acid, such as a TPP1 encoding nucleic acid sequence. Typically, for AAV one or both inverted terminal repeat (ITR) sequences of AAV genome are retained in the AAV vector. A "recombinant" viral vector (e.g., AAV) is distinguished from a viral (e.g., AAV) genome, since all or a part of the viral genome has been replaced with a non-native sequence with respect to the viral (e.g., AAV) genomic nucleic acid such as TPP1 encoding nucleic acid sequence. Incorporation of a non-native sequence therefore defines the viral vector (e.g., AAV) as a "recombinant" vector, which in the case of AAV can be referred to as a "rAAV vector."

An AAV vector (e.g., rAAV vector) can be packaged and is referred to herein as an "AAV particle" for subsequent infection (transduction) of a cell, *ex vivo, in vitro* or *in vivo.* Where a recombinant AAV vector is encapsulated or packaged into an AAV particle, the particle can also be referred to as a "rAAV particle". In certain embodiments an AAV particle is an rAAV particle. An AAV particle often comprises an AAV vector, or a portion thereof. An AAV particle can be one or more AAV particles (e.g., a plurality of AAV particles). AAV particles typically comprise proteins that encapsulate or package the AAV vector genome (e.g., capsid proteins).

Any suitable AAV particle (e.g., rAAV particle) can be used for a method or composition herein. An AAV particle, and/or genome comprised therein, can be derived from any suitable serotype or strain of AAV. An AAV particle, and/or genome comprised therein, can be derived from two or more serotypes or strains of AAV. Accordingly, an AAV can comprise proteins and/or nucleic acids, or portions thereof, of any serotype or strain of AAV, wherein the AAV particle is suitable for infection and/or transduction of a mammalian cell. Non-limiting examples of AAV serotypes include AAV1, AAV2, AAV3, AAV4, AAV5, AAV6, AAV7, AAV8, AAV9, AAV10, AAV11, AAV12, AAV-rh74, AAV-rh10 or AAV-2i8. In certain embodiments a plurality of AAV particles comprises particles of, or derived from, the same strain or serotype (or subgroup or variant). In certain embodiments a plurality of AAV particles comprise a mixture of two or more different AAV particles (e.g., of different serotypes and/or strains).

In certain embodiments, an AAV particle is not an AAV4 particle. In certain embodiments, an AAV particle is antigenically or immunologically distinct from AAV4. Distinctness can be determined by standard methods. For example, ELISA and Western blots can be used to determine whether a viral particle is antigenically or immunologically distinct from AAV4. Furthermore, in certain embodiments an AAV2 viral particle retains tissue tropism distinct from AAV4.

In certain embodiments a rAAV vector based upon a first serotype genome is identical to the serotype of one or more of the capsid proteins that package the vector. In certain embodiments a recombinant AAV vector genome can be based upon an AAV (e.g., AAV2) serotype genome distinct from the serotype of one or more of the AAV capsid proteins that package the vector. For example, the AAV vector genome can comprise AAV2 derived nucleic acids (e.g., ITRs), whereas at least one or more of the three capsid proteins are derived from an AAV1, AAV3, AAV4, AAV5, AAV6, AAV7, AAV8, AAV9, AAV10, AAV11, AAV12, Rh10, Rh74 or AAV-2i8 serotype or variant thereof.

Recombinant AAV vectors that include a polynucleotide that directs the expression of a polypeptide can be generated using suitable recombinant techniques known in the art (e.g., see Sambrook et al., 1989). Recombinant AAV vectors are typically packaged into transduction-competent AAV particles and propagated using an AAV viral packaging system. A transduction-competent AAV particle is capable of binding to and entering a mammalian cell and subsequently delivering a nucleic acid cargo (e.g., a heterologous gene) to the nucleus of the cell. Thus, an intact AAV particle that is transduction-competent is configured to transduce a mammalian cell. An AAV particle configured to transduce a mammalian cell is often not replication competent, and requires additional protein machinery to self-replicate. Thus an AAV particle that is configured to transduce a mammalian cell is engineered to bind and enter a mammalian cell and deliver a nucleic acid to the cell, wherein the nucleic acid for delivery is often positioned between a pair of AAV ITRs in the AAV genome.

In certain embodiments, suitable host cells for producing transduction-competent AAV particles include microorganisms, yeast cells, insect cells, and mammalian cells that can be, or have been, used as recipients of a heterologous AAV vectors. Cells from the stable human cell line, 293 (readily available through, e.g., the American Type Culture Collection under Accession Number ATCC CRL1573) can be used in the practice of the present disclosure. In certain embodiments a modified human embryonic kidney cell line (e.g., HEK293), which is transformed with adenovirus type-5 DNA fragments, and expresses the adenoviral E1a and E1b genes is used to generate recombinant AAV particles. The modified HEK293 cell line is readily transfected, and provides a particularly convenient platform in which to produce rAAV particles. Methods of generating high titer AAV particles capable of transducing mammalian cells are known in the art. For example, AAV particle can be made as set forth in Wright, 2008 and Wright, 2009.

In certain embodiments, AAV helper functions are introduced into the host cell by transfecting the host cell with an AAV helper construct either prior to, or concurrently with, the transfection of an AAV expression vector. AAV helper constructs are thus sometimes used to provide at least transient expression of AAV rep and/or cap genes to complement missing AAV functions necessary for productive AAV transduction. AAV helper constructs often lack AAV ITRs and can neither replicate nor package themselves. These constructs can be in the form of a plasmid, phage, transposon, cosmid, virus, or virion. A number of AAV helper constructs have been described, such as the commonly used plasmids pAAV/Ad and pIM29+45 which encode both Rep and Cap expression products. A number of other vectors are known which encode Rep and/or Cap expression products.

A method as described herein may comprise use of an AAV2 particle. In a particular aspect, an AAV2 particle is a recombinant AAV2 particle. In certain embodiments an AAV2 particle comprises an AAV2 capsid. In certain embodiments an AAV2 particle comprises one or more capsid proteins (e.g., VP1, VP2 and/or VP3) that are at least 60%, 65%, 70%, 75% or more identical, e.g., 80%, 85%, 85%, 87%, 88%, 89%, 90%, 91%, 92%, 93%, 94%, 95%, 96%, 97%, 98%, 99%, 99.1%, 99.2%, 99.3%, 99.4%, 99.5%, etc., up to 100% identical to a corresponding capsid protein of a native or wild-type AAV2 particle. In certain embodiments an AAV2 particle comprises VP1, VP2 and VP3 capsid proteins that are at least 75% or more identical, e.g., 80%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, etc., up to 100% identical to a corresponding capsid protein of a native or wild-type AAV2 particle. In certain embodiments, an AAV2 particle is a variant of a native or wild-type AAV2 particle. In some aspects, one or more capsid proteins of an AAV2 variant have 1, 2, 3, 4, 5, 5-10, 10-15, 15-20 or more amino acid substitutions compared to capsid protein(s) of a native or wild-type AAV2 particle.

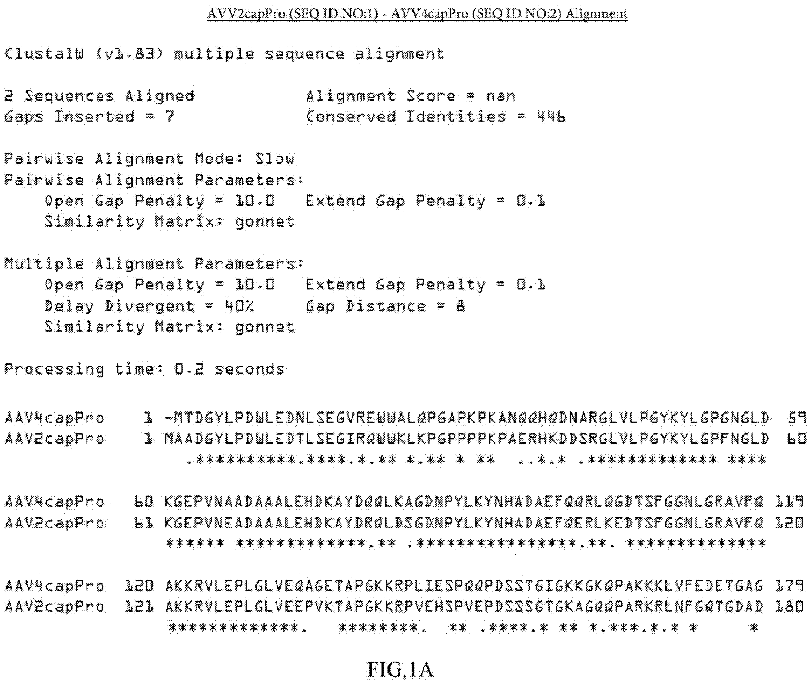

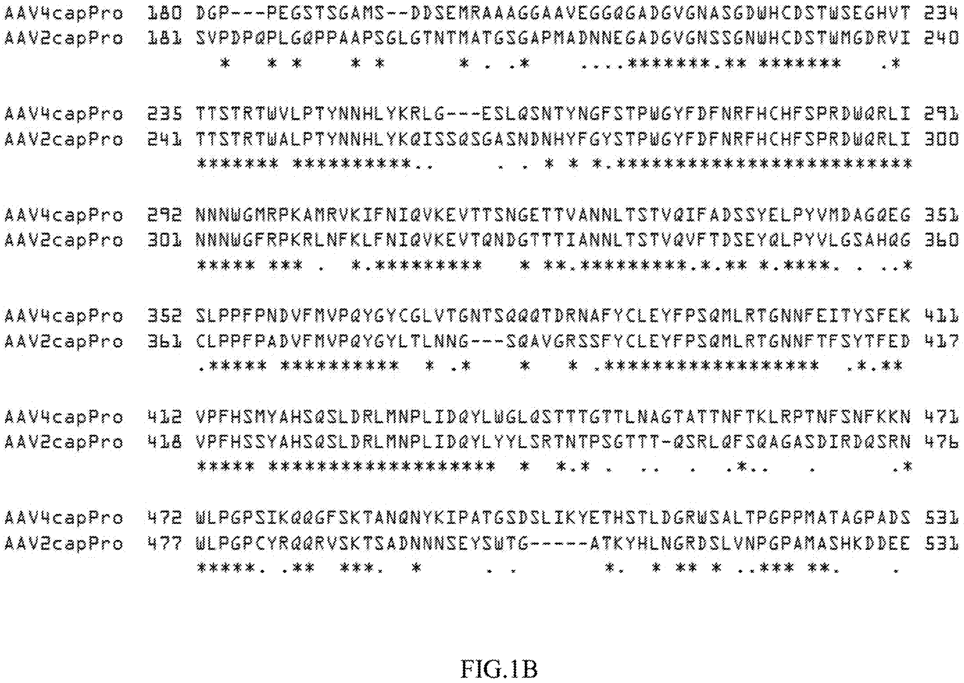

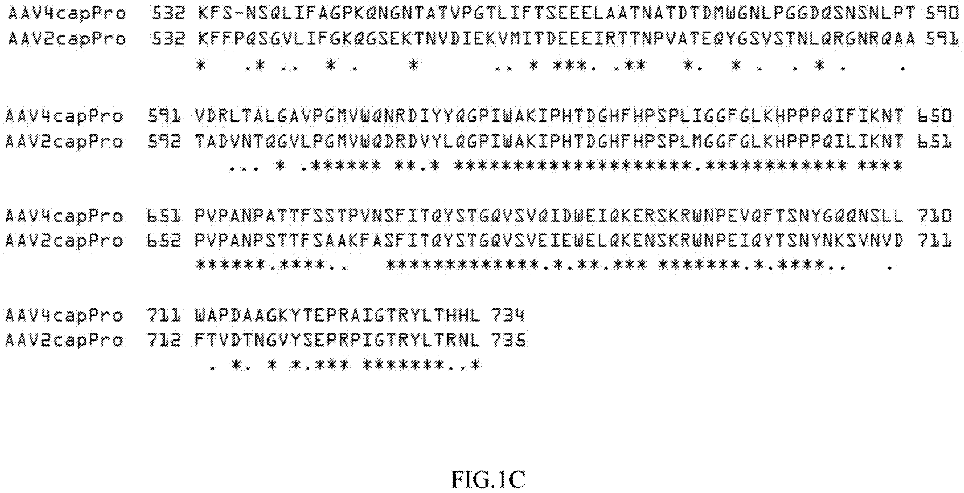

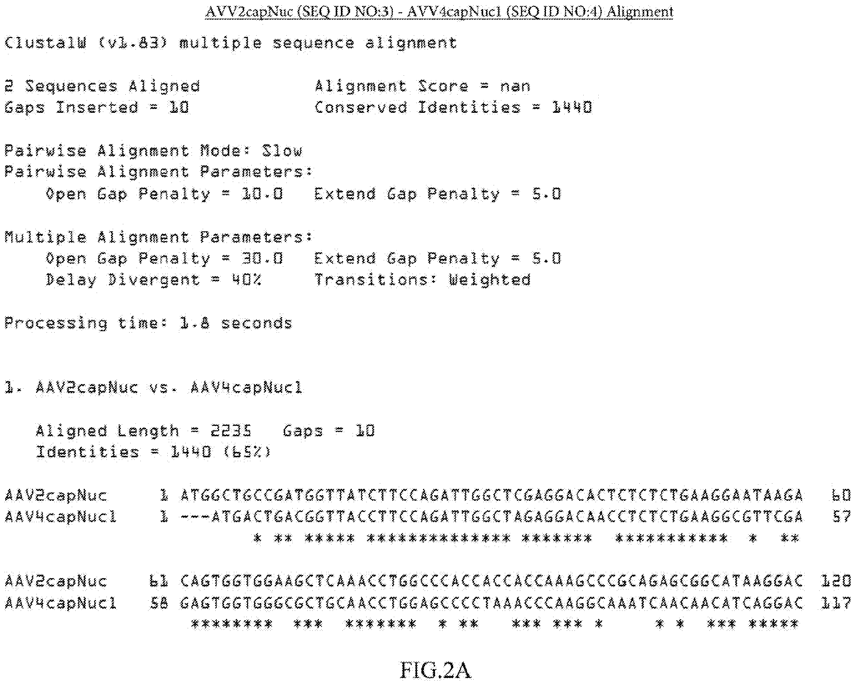

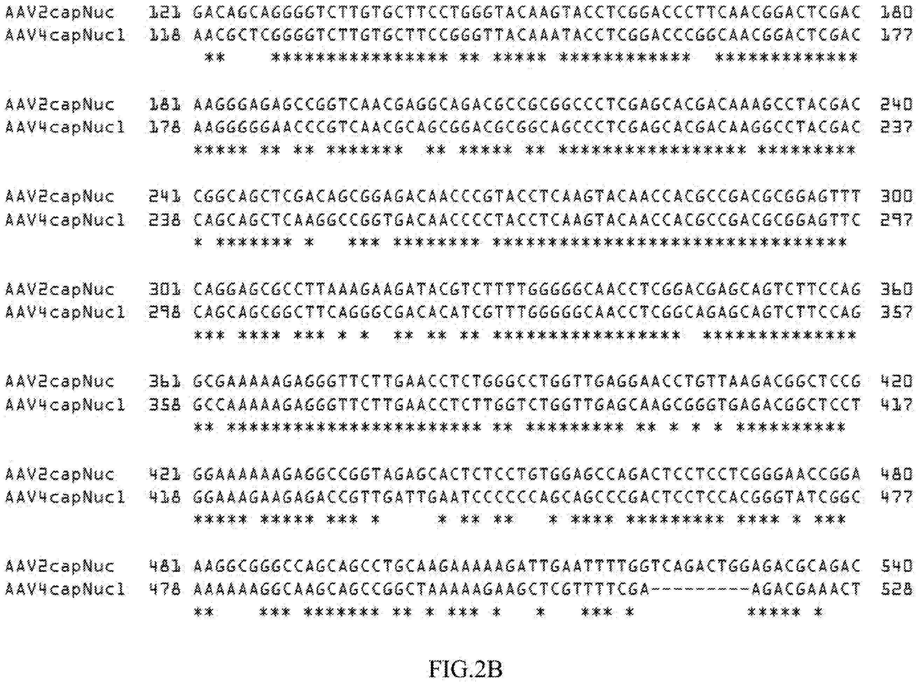

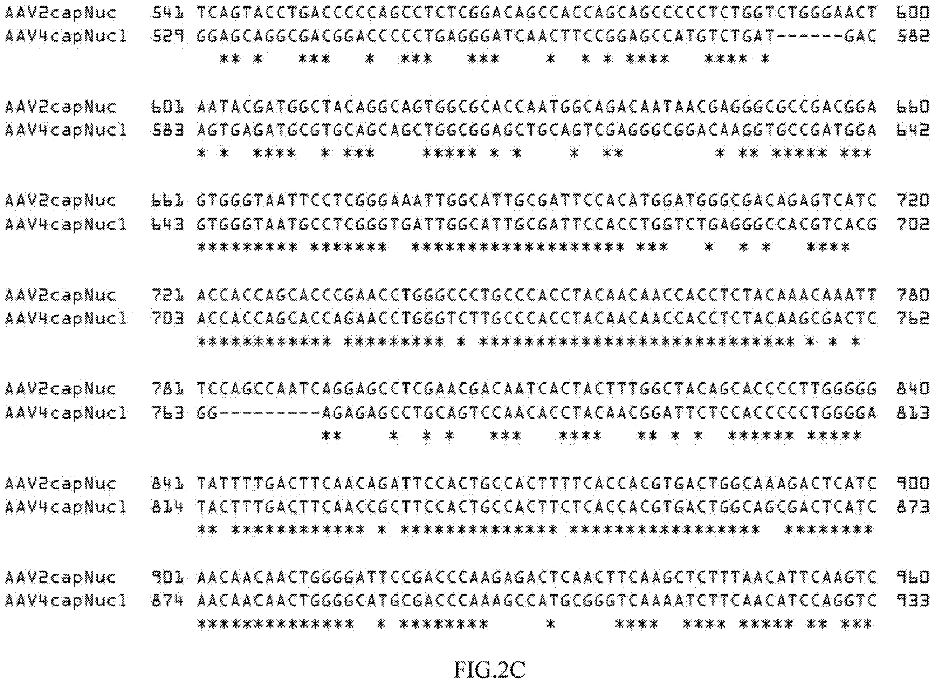

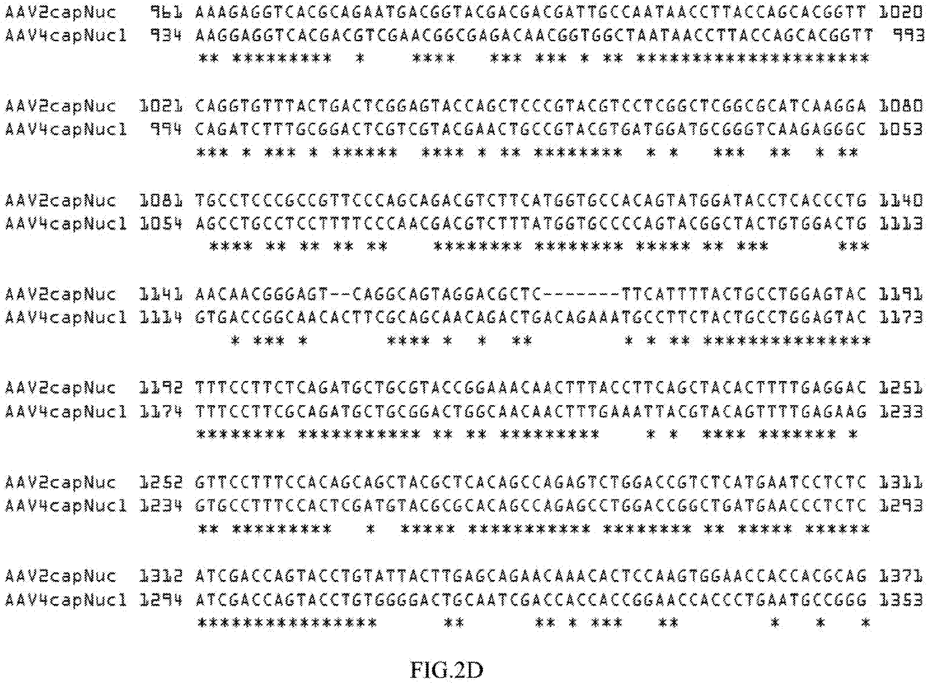

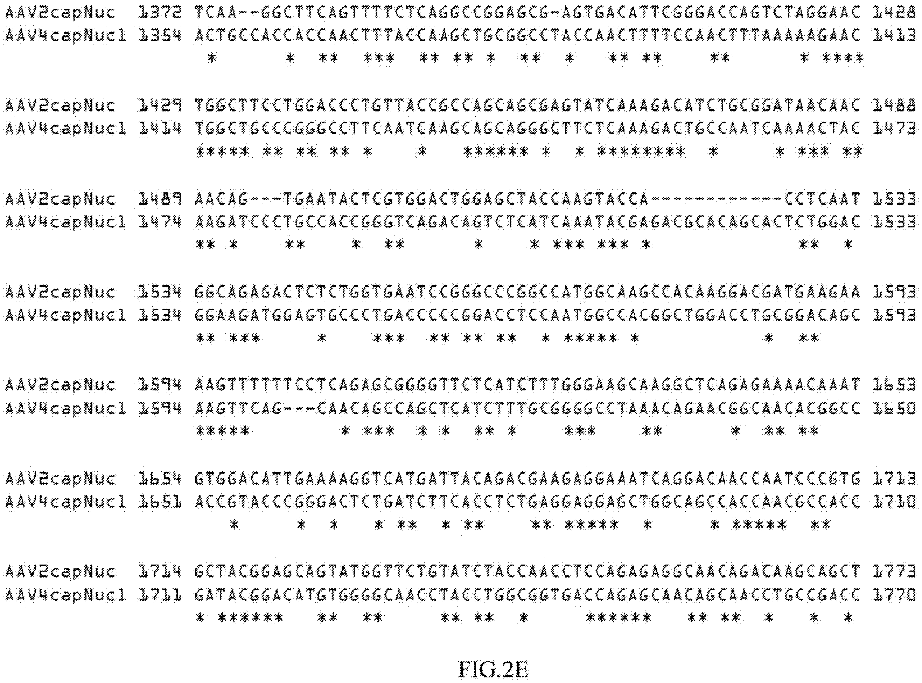

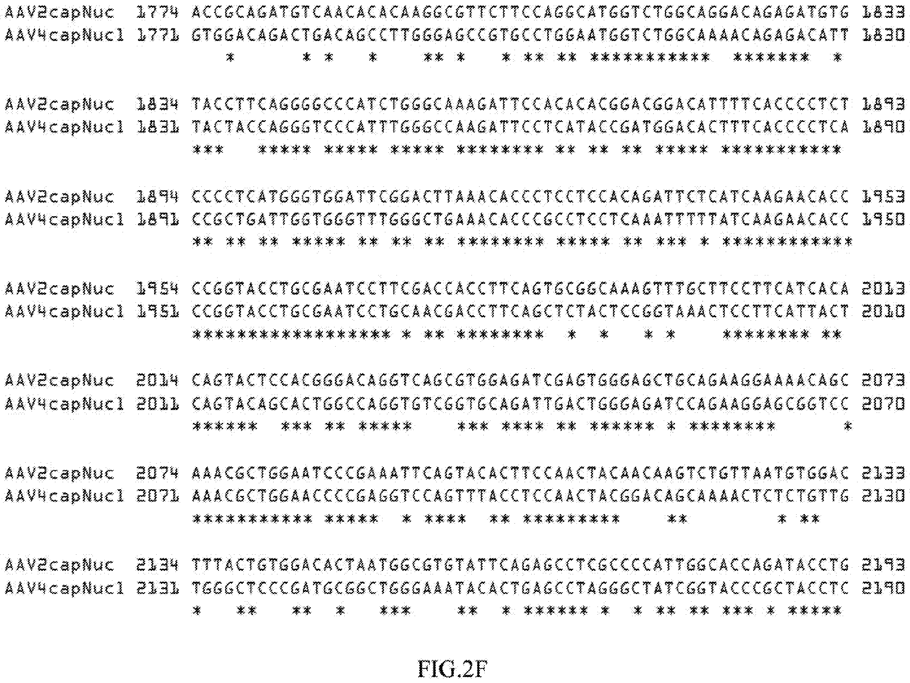

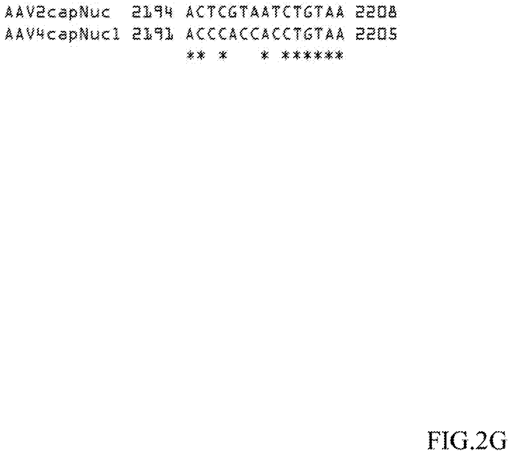

In certain embodiments an AAV2 particle (e.g., a capsid of an AAV2 particle) comprises a VP1 polypeptide having at least 60%, at least 70% identity, at least 75% identity, at least 80% identity, at least 85% identity, at least at least 90% identity, at least 95% identity, at least 98% identity, at least 99% identity, or even 100% identity to the protein set forth in SEQ ID NO:1. In certain embodiments an AAV2 particle comprises a VP1 polypeptide that is about 63% or more identical (e.g., 63% identity) to the polypeptide having the amino acid sequence of AAV2 VP1 capsid protein as in FIGS.1 and 2. AAV2 capsid sequence and AAV4 capsid sequence are about 60% homologous. In certain embodiments, the AAV2 VP1 capsid protein has a sequence that is at least 65% homologous to the amino acid sequence set forth in SEQ ID NO:1. In certain embodiments, the AAV2 VP1 capsid protein comprises the amino acid sequence set forth in SEQ ID NO:1.

In certain embodiments an AAV particle comprises one or two ITRs (e.g., a pair of ITRs) that are at least 75% or more identical, e.g., 80%, 85%, 85%, 87%, 88%, 89%, 90%, 91%, 92%, 93%, 94%, 95%, 96%, 97%, 98%, 99%, 99.1%, 99.2%, 99.3%, 99.4%, 99.5%, etc., up to 100% identical to corresponding ITRs of a native or wild-type AAV1, AAV2, AAV3, AAV4, AAV5, AAV6, AAV7, AAV8, AAV9, AAV10, AAV11, AAV12, AAV-rh74, AAV-rh10 or AAV-2i8, as long as they retain one or more desired ITR functions (e.g., ability to form a hairpin, which allows DNA replication; integration of the AAV DNA into a host cell genome; and/or packaging, if desired). In other embodiments an AAV2 particle comprises one or two ITRs (e.g., a pair of ITRs) that are at least 75% or more identical, e.g., 80%, 85%, 85%, 87%, 88%, 89%, 90%, 91%, 92%, 93%, 94%, 95%, 96%, 97%, 98%, 99%, 99.1%, 99.2%, 99.3%, 99.4%, 99.5%, etc., up to 100% identical to corresponding ITRs of a native or wild-type AAV2 particle, as long as they retain one or more desired ITR functions (e.g., ability to form a hairpin, which allows DNA replication; integration of the AAV DNA into a host cell genome; and/or packaging, if desired).

An AAV2 particle can comprise an ITR having any suitable number of "GAGC" repeats. In certain embodiments an ITR of an AAV2 particle comprises 1, 2, 3, 4, 5, 6, 7, 8, 9 or 10 or more "GAGC" repeats. In certain embodiments an AAV2 particle comprises an ITR comprising three "GAGC" repeats. In certain embodiments an AAV2 particle comprises an ITR which has less than four "GAGC" repeats. In certain embodiments an AAV2 particle comprises an ITR which has more than four "GAGC" repeats. In certain embodiments an ITR of an AAV2 particle comprises a Rep binding site wherein the fourth nucleotide in the first two "GAGC" repeats is a C rather than a T.

Any suitable length of DNA can be incorporated into an AAV2 particle. Suitable DNA molecules for use in AAV vectors can about 5 kilobases (kb), less than about 5kb, less than about 4.5 kb, less than about 4 kb, less than about 3.5 kb, less than about 3 kb, or less than about 2.5 kb.

TPP1 is a lysosomal serine protease encoded by the CLN2 gene (TPP1 gene). The amino acid sequence of human TPP1 is set forth as SEQ ID NO:5. The nucleic acid sequence of human TPP1 is set forth as SEQ ID NO:6. Human TPP1 comprises tripeptidyl-peptidase I activity (TPP1 enzyme activity). TPP1 activity comprises a non-specific lysosomal peptidase activity which generates tripeptides from the breakdown products produced by lysosomal proteinases. Substrate-specificity studies indicate that TPP1 primarily cleaves tripeptides from unsubstituted amino termini in peptides and proteins. Endogenously expressed TPP1 is synthesized as a catalytically-inactive enzyme. After targeting into lysosomes, because of the acidic environment, the TPP1 is auto-catalytically processed into a mature active enzyme. The activity of TPP1 can be measured and/or quantitated *in vitro* using known methods. See, for example, Junaid et al., 1999.

A polypeptide comprising TPP1 activity refers to a TPP1 protein of a mammal, or a portion thereof, that displays at least 50%, at least 60%, at least 70%, at least 75%, at least 80%, at least 85%, at least 90%, at least 95%, or about 100% of the peptidase activity of the human TPP1 of SEQ ID NO:5 as assayed using a suitable peptide substrate, for example, as assayed by the method of Junaid et al., 1999 or another comparable method. A polypeptide comprising TPP1 activity refers to a TPP1 protein of a mammal, or a subsequence or variant thereof, that displays at least at least 50%, at least 60%, at least 70%, at least 75%, at least 80%, at least 85%, at least 90%, at least 95%, or about 100% of the peptidase activity of the human TPP1 of SEQ ID NO: 5.

A polypeptide comprising TPP1 activity may comprise a truncated, mutated, chimeric, or modified form of a TPP1 polypeptide that retains at least partial TPP1 activity. A polypeptide comprising TPP1 activity may comprise a TPP1 protein, or a portion thereof, obtained from any suitable organism (e.g., from a mammal, from a human, from a non-human mammal, e.g., from a dog, pig, cow, or the like). In certain embodiments a polypeptide comprising TPP1 activity has at least 60% identity, at least 70% identity, at least 75% identity, at least 80% identity, at least 85% identity, at least 90% identity, at least 95% identity, at least 98% identity, or 100% identity to the TPP1 protein set forth in SEQ ID NO:5. According to the claimed invention, the polypeptide comprises a TPP1 polypeptide having at least 90% identity to an amino acid sequence set forth in SEQ ID NO:5 or a pro-enzyme thereof.

An AAV particle comprises an AAV capsid protein and a nucleic acid encoding a polypeptide comprising TPP1 activity. In certain embodiments an AAV particle comprises an AAV capsid protein and a nucleic acid that directs the expression and/or secretion of a polypeptide comprising TPP1 activity. According to the claimed invention, the nucleic acid encodes a polypeptide, wherein said polypeptide comprises a TPP1 polypeptide having at least 90% identity to an amino acid sequence set forth in SEQ ID NO:5 or a pro-enzyme thereof.

In certain embodiments an AAV particle comprises an AAV capsid protein and a nucleic acid encoding a TPP1 polypeptide, or enzymatically active portion thereof. In certain embodiments an AAV particle comprises an AAV capsid protein and a nucleic acid that directs the expression and/or secretion of a TPP1 polypeptide, or enzymatically active portion thereof. In certain embodiments, a nucleic acid being administered encodes TPP1, a TPP1 that has substantial identity to wild type TPP1, and/or a variant, mutant or fragment of a TPP1. In certain embodiments a TPP1 polypeptide has at least 60% identity, at least 70% identity, at least 75% identity, at least 80% identity, at least 85% identity, at least 90% identity, at least 95% identity, at least 98% identity, or 100% identity to the protein set forth in SEQ ID NO:5. In other embodiments a TPP1 polypeptide has at least 60% identity, at least 70% identity, at least 75% identity, at least 80% identity, at least 85% identity, at least 90% identity, at least 95% identity, at least 98% identity, or 100% identity to the protein set forth in SEQ ID NOs:7 or 8. According to the claimed invention, the nucleic acid encodes a polypeptide, wherein said polypeptide comprises a TPP1 polypeptide having at least 90% identity to an amino acid sequence set forth in SEQ ID NO:5 or a pro-enzyme thereof.

In certain embodiments an AAV particle comprises a nucleic acid having at least 50% identity, at least 60% identity, at least 70% identity, at least 75% identity, at least 80% identity, at least 85% identity, at least 90% identity, at least 95% identity, at least 98% identity, or 100% identity to the nucleic acid set forth in SEQ ID NO:6. In certain embodiments a nucleic acid encoding a TPP1 activity or encoding or directing the expression of a TPP1 polypeptide is a nucleic acid having at least 50% identity, at least 60% identity, at least 70% identity, at least 75% identity, at least 80% identity, at least 85% identity, at least 90% identity, at least 95% identity, at least 98% identity, or 100% identity to the nucleic acid set forth in SEQ ID NO:6.

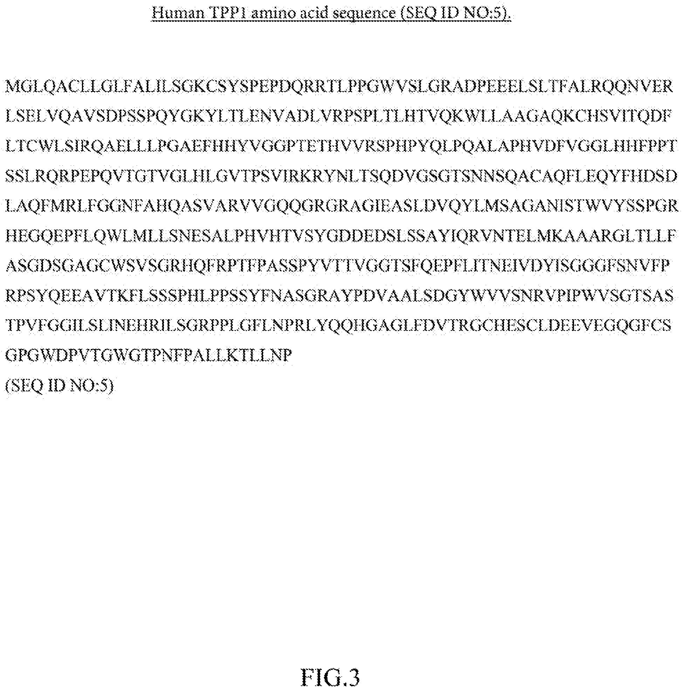

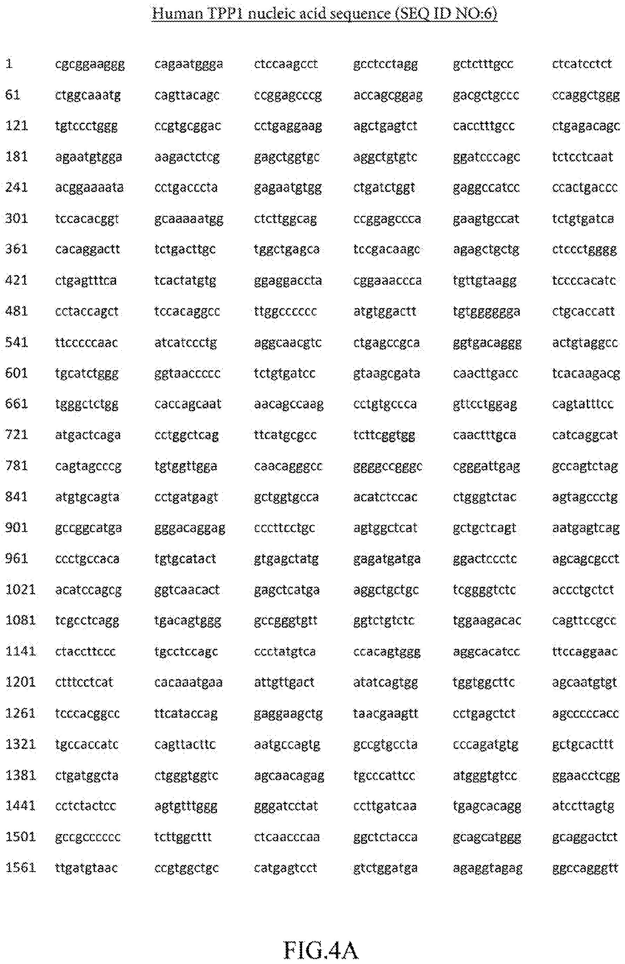

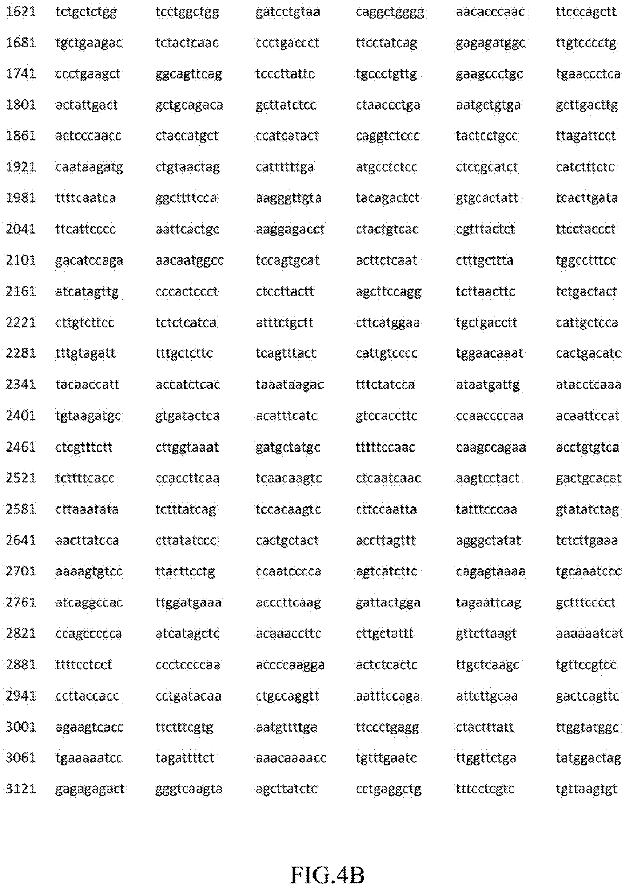

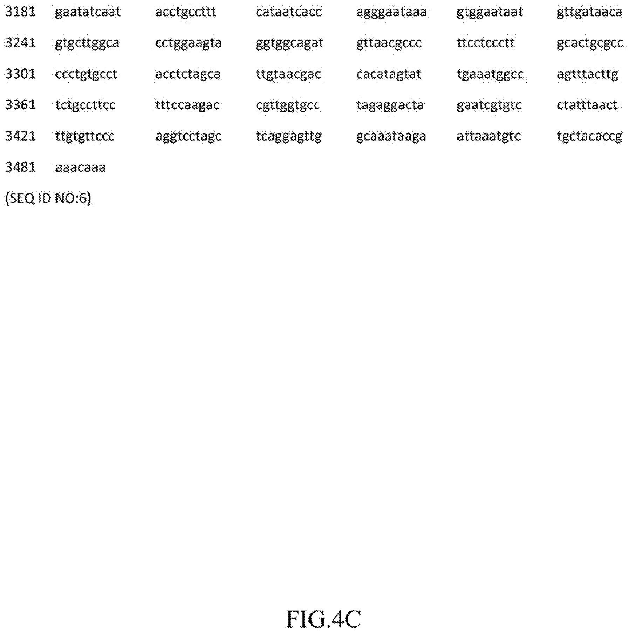

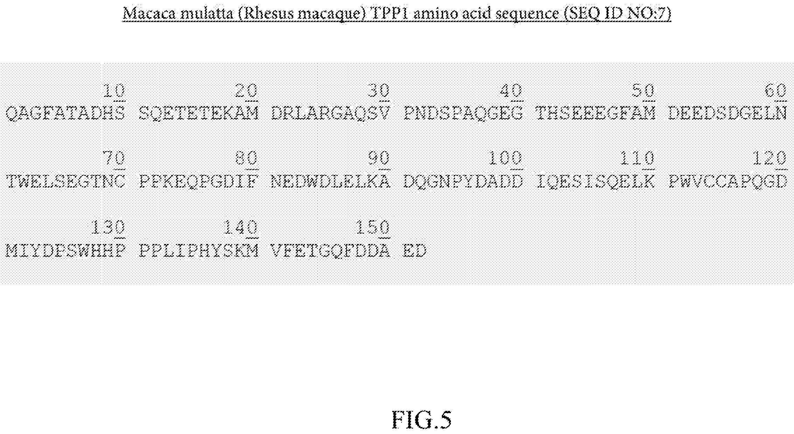

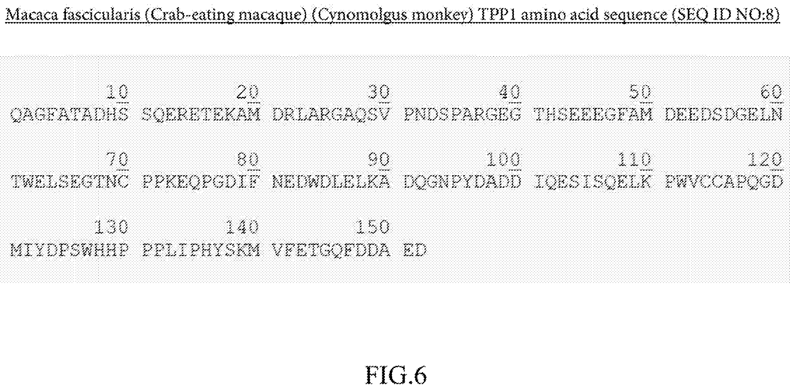

A human TPP1 amino acid sequence is depicted in FIG.3 (SEQ ID NO:5), A human TPP1 nucleic acid sequence is depicted in FIGS.4A-6C (SEQ ID NO:6). FIG.5 depicts the *Macaca mulatta* TPP1 amino acid sequence (SEQ ID NO:7), and FIG.6 depicts the *Macaca fascicularis* TPP1 amino acid sequence (SEQ ID NO:8).

In certain embodiments a method described herein includes administering or delivering AAV-TPP1 particles to a mammal and administering one or more immunosuppressive agents to the mammal. In certain embodiments a method described herein includes administering or delivering AAV-TPP1 particles to a mammal and administering 2, 3, 4 or more immunosuppressive agents to the mammal. In certain embodiments a method described herein includes administering or delivering AAV-TPP1 particles to a mammal and administering two immunosuppressive agents to the mammal. In one representative embodiment, a method of treating a mammal as described herein includes administering or delivering AAV-TPP1 particles to a mammal and administering first and second immunosuppressive agents to the mammal.

Where two or more immunosuppressive agents are administered, each immunosuppressive agent is distinct and/or different (e.g., each agent differs in structure and/or mechanism of action). An "agent" refers to an active pharmaceutical ingredient. In certain embodiments, an immunosuppressive agent is an anti-inflammatory agent. In certain embodiments, an immunosuppressive agent is mycophenolate, or a derivative thereof. An example of such a mycophenolate derivative is mycophenolate mofetil (MMF). In certain embodiments, an immunosuppressive agent is cyclosporine or a derivative thereof. In certain embodiments a first immunosuppressive agent comprises cyclosporine and a second immunosuppressive agent comprises mycophenolate, or a derivative thereof (e.g., MMF). In certain embodiments a first immunosuppressive agent comprises cyclosporine and a second immunosuppressive agent comprises MMF. According to the claimed invention, the first immunosuppressive agent is cyclosporine and the second immunosuppressive agent is mycophenolate mofetil (MMF).

In certain embodiments, an immunosuppressive agent is administered before, during and/or after administration of AAV-TPP1 particles to a mammal. In certain embodiments, an immunosuppressive agent is administered concurrently with administration of AAV-TPP1 particles to a mammal. In certain embodiments, an immunosuppressive agent is administered after administration of AAV-TPP1 particles to a mammal. In certain embodiments, an immunosuppressive agent is administered about 1 to about 60 minutes after, about 1 to about 24 hours after, about 1 to about 100 days after, about 1 to about 12 months after, or about 1 to about 5 years after administration of AAV-TPP1 particles to a mammal. In certain embodiments, an immunosuppressive agent is administered before administration of AAV-TPP1 particles to a mammal. In certain embodiments, an immunosuppressive agent is administered about 1 to about 60 minutes before, about 1 to about 24 hours before, about 1 to about 100 days before, or about 1 to about 3 months before administration of AAV-TPP1 particles to a mammal. In certain embodiments, an immunosuppressive agent is administered about 1, about 2, about 3, about 4, about 5, about 6, about 7, about 8, about 9 or about 10 days before administration of AAV-TPP1 particles to a mammal. In certain embodiments, an immunosuppressive agent is administered at predetermined intervals before, during and/or after administration of AAV-TPP1 particles to a mammal (e.g., once a day, twice a day, three times a day, every other day, weekly, biweekly, bi-monthly, combination thereof or the like). According to the claimed invention, at least one of the first immunosuppressive agent and the second immunosuppressive agent are to be administered to the mammal prior to administration of the AAV particles.

In certain embodiments a first immunosuppressive agent is administered to a mammal at least about 1 to about 7 days before, or about 1, about 2, about 3, about 4 or about 5 weeks before administration of AAV-TPP1 particles to a mammal and a second immunosuppressive agent is administered about 1 to about 7 days before, about 1, about 2, about 3, about 4 or about 5 weeks before, during and/or within about 10, about 20, about 30, about 40, about 50, about 100, about 200, about 300, about 350, about 400 or about 500 days after administration of AAV-TPP1 particles to the mammal. In certain embodiments cyclosporine is administered to a mammal at least about 1 to about 7 days before, or about 1, about 2, about 3, about 4 or about 5 weeks before administration of AAV-TPP1 particles to a mammal, and mycophenolate or a derivative thereof (e.g., MMF) is administered about 1 to about 7 days before, about 1, about 2, about 3, about 4 or about 5 weeks before, during and/or within about 10, about 20, about 30, about 40, about 50, about 100, about 200, about 300, about 350, about 400 or about 500 days after administration of AAV-TPP1 particles to the mammal. In certain embodiments, cyclosporine is administered about 1 to about 7 days before, or about 1, about 2, about 3, about 4 or about 5 weeks before administration of AAV-TPP1 particles and at regular intervals after treatment, and mycophenolate or a derivative thereof (e.g., MMF) is administered once at about 1 to about 7 days before, about 1, about 2, about 3, about 4 or about 5 weeks before, during and/or within about 10 to about 40 days after administration of AAV-TPP1 particles to the mammal. According to the claimed invention, the first immunosuppressive agent is cyclosporine and the second immunosuppressive agent is MMF.

An immunosuppressive agent can be administered at any suitable dose. In certain embodiments, cyclosporine is administered at a dosage of about 1 to about 50 mg/kg, about 1 to about 20 mg/kg, or about 5 to about 10 mg/kg at a frequency of once, twice or three times a day, to once every other day. In certain embodiments cyclosporine is administered at about 10 mg/kg twice a day. In certain embodiments, cyclosporine is administered at about 10 mg/kg twice a day for a period of at least about 1, about 2, about 3, about 4 or about 5 months. In certain embodiments, a dosage of cyclosporine is tapered down to a dose of less than about 5 mg/kg, or less than about 2 mg/kg about 1 to about 2 months after the administration of AAV-TPP1 particles to a mammal.

In certain embodiments, mycophenolate or a derivative thereof (e.g., MMF), is administered at a dosage of about 1 to about 100 mg/kg, about 1 to about 50 mg/kg, about 1 to about 25 mg/kg, or about 5 to about 20 mg/kg at a frequency of once, twice or three times a day, to once every other day. In certain embodiments, mycophenolate or a derivative thereof (e.g., MMF) is administered at about 10 to about 20 mg/kg once a day. In certain embodiments, a dosage of mycophenolate or a derivative thereof (e.g., MMF) is reduced down to a dose of less than about 5 mg/kg, or less than about 2 mg/kg about 1 to about 2 months after the administration of AAV-TPP1 particles to a mammal. According to the claimed invention, the first immunosuppressive agent is cyclosporine and the second immunosuppressive agent is MMF.

An immunosuppressive agent can be formulated in any suitable formulation suitable for a particular route of administration. Various pharmaceutically acceptable formulations of immunosuppressive agents are commercially available and readily obtainable by a medical practitioner.

An immunosuppressive agent can be administered by any suitable route. In certain embodiments, an immunosuppressive agent is administered orally. In certain embodiments, mycophenolate or a derivative thereof, such as Mycophenolate Mofetil (MMF), is administered orally. In certain embodiments, cyclosporine is administered orally. An immunosuppressive agent can also be administered parenterally (e.g., intramuscularly, intravenously, subcutaneously), or administered by injection to the brain, spinal cord, or a portion thereof (e.g., injected into the CSF). According to the claimed invention, the first immunosuppressive agent is cyclosporine and the second immunosuppressive agent is MMF.

In certain embodiments a method described herein includes administering one or more (e.g., a plurality of) AAV-TPP1 particles to the central nervous system of a mammal (e.g., a mammal having a LSD). In certain embodiments, the central nervous system includes brain, spinal cord and cerebral spinal fluid (CSF). In certain embodiments, a method described herein includes administering one or more AAV-TPP1 particles to the brain or spinal cord or CSF of a mammal. In certain embodiments AAV-TPP1 particles are administered to a portion of brain or spinal cord. In certain embodiments, a composition including AAV-TPP1 particles and an immunosuppressive agent are administered to a mammal's cisterna magna and/or to the mammal's brain ventricle, subarachnoid space, and/or intrathecal space, and/or ependyma. For example, AAV-TPP1 particles can be delivered directly to the cisterna magna, intraventricular space, a brain ventricle, subarachnoid space, intrathecal space or ependyma. In certain embodiments a method described herein includes administering one or more AAV-TPP1 particles to the ependyma of a mammal.

In certain embodiments AAV-TPP1 particles are administered to one or more cells that contact the CSF in a mammal, for example by contacting cells with AAV-TPP1 particles. Non-limiting examples of cells that contact the CSF include ependymal cells, pial cells, endothelial cells and/or meningeal cells. In certain embodiments AAV-TPP1 particles are administered to ependymal cells. In certain embodiments AAV-TPP1 particles are delivered to ependymal cells, for example by contacting ependymal cells with AAV-TPP1 particles.

In certain embodiments AAV-TPP1 particles are delivered locally. "Local delivery" refers to delivery of an active agent directly to a target site within a mammal (e.g., directly to a tissue or fluid). For example, an agent can be locally delivered by direct injection into an organ, tissue or specified anatomical location. In certain embodiments one or more AAV-TPP1 particles are delivered or administered by direct injection to the brain, spinal cord, or a tissue or fluid thereof (e.g., CSF, such as ependymal cells, pial cells, endothelial cells and/or meningeal cells). For example AAV-TPP1 particles can be directly delivered, by way of direct injection, to the CSF, cisterna magna, intraventricular space, a brain ventricle, subarachnoid space and/or intrathecal space and/or ependyma. In certain embodiments AAV-TPP1 particles are contacted with a tissue, fluid or cell of the brain or spinal cord by direct injection into a tissue or fluid of the brain or spinal cord. In certain embodiments AAV-TPP1 particles are not delivered systemically by, for example, intravenous, subcutaneous, or intramuscular injection, or by intravenous infusion. In certain embodiments AAV-TPP1 particles are delivered to a tissue or fluid of the brain or spinal cord by stereotactic injection.

In certain embodiments one or more AAV-TPP1 particles are delivered or administered by direct injection of AAV-TPP1 particles to the brain, spinal cord, or a tissue or fluid thereof (e.g., CSF such as ependyma). In a particular aspect, AAV-TPP1 particles transduce ependymal cells, pial cells, endothelial cells and/or meningeal cells.

As is apparent to those skilled in the art in view of the teachings herein, such as the dose ranges provided herein, an effective amount of AAV-TPP1 particles can be empirically determined. Administration can be effected in one dose, continuously or intermittently throughout the course of treatment. Effective doses of administration can be determined by those of skill in the art and may vary according to the AAV serotype, viral titer and the weight, condition and species of mammal being treated. Single and multiple administrations can be carried out with the dose level, target and timing being selected by the treating physician.

In certain embodiments, a plurality of AAV-TPP1 particles are administered. As used herein, a plurality of AAV particle refers to about 1×10⁵ to about 1×10¹⁸ particles.

In certain embodiments, AAV-TPP1 particles are administered at a dose of about 1×10⁵ to about 1×10¹⁶ vg/ml in about 1 to about 5 ml; at a dose of about 1 to about 3 ml of 1×10⁷ to about 1×10¹⁴ vg/ml; or at a dose of about 1 to about 2 ml of 1×10⁸ to about 1×10¹³ vg/ml. In certain embodiments, AAV-TPP1 particles are administered at a dose of about 1×10⁸ to about 1×10¹⁵ vg/kg body weight of the mammal being treated. For example, AAV-TPP1 particles can be administered at a dose of about 1×10⁸ vg/kg, about 5×10⁸ vg/kg, about 1×10⁹ vg/kg, about 5×10⁹ vg/kg, about 1×10¹⁰ vg/kg, about 5×10¹⁰ vg/kg, about 1×10¹¹ vg/kg, about 5×10¹¹ vg/kg, about 1×10¹² vg/kg, about 5×10¹² vg/kg, about 1×10¹³ vg/kg, about 5×10¹³ vg/kg, about 1×10¹⁴ vg/kg, about 5×10¹⁴ vg/kg, or about 1×10¹⁵ vg/kg body weight of the mammal being treated.

Administration of AAV-TPP1 particles may be in one or more doses. Multiple doses may be administered as is required to maintain adequate enzyme activity, for example.

Pharmaceutical forms suitable for injection or infusion of AAV-TPP1 particles can include sterile aqueous solutions or dispersions which are adapted for the extemporaneous preparation of sterile injectable or infusible solutions or dispersions, optionally encapsulated in liposomes. In all cases, the ultimate form should be a sterile fluid and stable under the conditions of manufacture, use and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. Isotonic agents, for example, sugars, buffers or salts (e.g., sodium chloride) can be included. Prolonged absorption of injectable compositions can be brought about by the use in the compositions of agents delaying absorption, for example, aluminum monostearate and gelatin.

Solutions or suspensions of AAV-TPP1 particles can optionally include the following components: a sterile diluent such as water for injection, saline solution, such as phosphate buffered saline (PBS), artificial CSF, fixed oils, a polyol (for example, glycerol, propylene glycol, and liquid polyethylene glycol, and the like), glycerin, or other synthetic solvents; antibacterial and antifungal agents such as parabens, chlorobutanol, phenol, ascorbic acid, and the like; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose.

AAV-TPP1 particles and compositions may be formulated in dosage unit form for ease of administration and uniformity of dosage. Dosage unit form as used herein refers to physically discrete units suited as unitary dosages for an individual to be treated; each unit containing a predetermined quantity of active compound calculated to produce the desired therapeutic effect in association with the required pharmaceutical carrier. The dosage unit forms are dependent upon the amount of AAV-TPP1 particles necessary to produce the desired effect(s). The amount necessary can be formulated in a single dose, or can be formulated in multiple dosage units. The dose may be adjusted to a suitable AAV-TPP1 particles concentration, optionally combined with an anti-inflammatory agent, and packaged for use.

In one embodiment, pharmaceutical compositions will include sufficient genetic material to provide a therapeutically effective amount, i.e., an amount sufficient to reduce or ameliorate symptoms of a disease state in question or an amount sufficient to confer the desired benefit. Pharmaceutical compositions typically contain a pharmaceutically acceptable excipient. Such excipients include any pharmaceutical agent that does not itself induce the production of antibodies harmful to the individual receiving the composition, and which may be administered without undue toxicity. Pharmaceutically acceptable excipients include, but are not limited to, sorbitol, Tween80, and liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable excipients is available in Remington's Pharmaceutical Sciences, 1991.

Formulations containing AAV-TPP1 particles will contain an effective amount of the rAAV particles in a vehicle, the effective amount being readily determined by one skilled in the art. The AAV-TPP1 particles may typically range from about 1% to about 95% (w/w) of the composition, or even higher if suitable. The quantity to be administered depends upon factors such as the age, weight and physical condition of the mammal or the human subject considered for treatment. Effective dosages can be established by one of ordinary skill in the art through routine trials establishing dose response curves.

In certain embodiments a method described herein includes administering a plurality of AAV-TPP1 particles a mammal (e.g., a mammal having an LSD) as set forth herein, optionally with a first and a second immunosuppressive agent, where time of onset of one or more symptoms of a LSD are delayed. The term "time of onset" refers to a point in time after a first administration of AAV-TPP1 particles that a symptom of LSD is first observed or detected. Non-limiting symptoms of LSD whose onset may be delayed include a proprioceptive response, nystagmus, menace, pupillary light reflex, cerebellar ataxia and intention tremor. The time of onset of a symptom (e.g., a sign of neurologic dysfunction) can be subjectively determined by a standardized clinical neurologic examination (e.g., see Lorenz et al., 2011).

A delay in the time of onset of a symptom associated with LSD can be determined by comparing the time of onset of a symptom for a mammal treated with AAV-TPP1 particles, a first and a second immunosuppressive agent (e.g., cyclosporine and MMF) to one or more mammals treated with AAV-TPP1 particles and mycophenolate (e.g., in the absence of a first immunosuppressive agent such as cyclosporine). In certain embodiments a method described herein includes administering a plurality of AAV-TPP1 particles to the central nervous system, or portion thereof, of a mammal (e.g., a mammal having an LSD) with a first and a second immunosuppressive agent where a time of onset of one or more symptoms of a LSD are delayed by at least about 5 to about 10, about 10 to about 25, about 25 to about 50, or about 50 to about 100 days compared to one or more mammals treated with AAV-TPP1 particles and mycophenolate (e.g., in the absence of a first immunosuppressive agent such as cyclosporine).

In certain embodiments a method described herein includes administering a plurality of AAV-TPP1 particles to the central nervous system, or portion thereof, of a mammal (e.g., a mammal having an LSD) with a first and a second immunosuppressive agent where a time of onset of a loss of cognitive function is delayed by at least about 5 to about 10, about 10 to about 25, about 25 to about 50 or about 50 to about 100 days compared to one or more mammals treated with AAV-TPP1 particles and mycophenolate (e.g., in the absence of a first immunosuppressive agent such as cyclosporine). The time of onset of a loss of cognitive function can be determined by Tmaze (Sanders et al., 2011). In certain embodiments a method described herein comprises administering a plurality of AAV-TPP1 particles to the central nervous system, or portion thereof, of a mammal (e.g., a mammal having an LSD) with a first and a second immunosuppressive agent where lifespan of the mammal is extended by at least about 5 to about 10, about 10 to about 25, about 25 to about 50 or about 50 to about 100 days compared to one or more mammals treated with AAV-TPP1 particles and mycophenolate (e.g., in the absence of a first immunosuppressive agent such as cyclosporine).

In certain embodiments a method described herein includes administering a plurality of AAV-TPP1 particles to the central nervous system, or portion thereof, of a mammal (e.g., a mammal having an LSD) with a first and a second immunosuppressive agent where the presence of neutralizing antibodies is not detected in CSF of the mammal for at least about 10 to at least about 300 days after administering the AAV-TPP1 particles. In certain embodiments a method described herein includes administering a plurality of AAV-TPP1 particles to the central nervous system, or portion thereof, of a mammal (e.g., a mammal having an LSD) with a first and a second immunosuppressive agent where neutralizing antibodies are not detected in CSF of the mammal for at least about 20 days, at least about 30 days, at least about 40 days, at least about 50 days, at least about 60 days, at least about 70 days, at least about 80 days, at least about 90 days, at least about 100 days, at least about 150 days, at least about 200 days, at least about 250 days, or at least, at least about 300 days after administering the AAV-TPP1 particles.

In certain embodiments a method described herein includes administering a plurality of AAV particles to the brain or spinal cord, or portion thereof, of a mammal where the AAV particles are configured to transduce cells of the mammal and direct expression of a polypeptide having TPP1 activity in the mammal, where the polypeptide is expressed and/or detected in one or more peripheral organs (e.g., in spleen and/or heart).

In certain embodiments a method described herein includes administering a plurality of AAV particles to the brain or spinal cord, or portion thereof, of a mammal where the AAV particles are configured to transduce cells of the mammal and direct expression of the polypeptide having TPP1 activity in the mammal, where the polypeptide is expressed and/or detected in a central nervous tissue (e.g., brain, e.g., cerebellum).

The terms "polynucleotide" and "nucleic acid" are used interchangeably herein to refer to all forms of nucleic acid, oligonucleotides, including deoxyribonucleic acid (DNA) and ribonucleic acid (RNA) and polymers thereof. Polynucleotides include genomic DNA, cDNA and antisense DNA, and spliced or unspliced mRNA, rRNA, tRNA and inhibitory DNA or RNA (RNAi, e.g., small or short hairpin (sh)RNA, microRNA (miRNA), small or short interfering (si)RNA, trans-splicing RNA, or antisense RNA). Polynucleotides can include naturally occurring, synthetic, and intentionally modified or altered polynucleotides (e.g., variant nucleic acid). Polynucleotides can be single stranded, double stranded, or triplex, linear or circular, and can be of any suitable length. In discussing polynucleotides, a sequence or structure of a particular polynucleotide may be described herein according to the convention of providing the sequence in the 5' to 3' direction.

A nucleic acid encoding a polypeptide often comprises an open reading frame that encodes the polypeptide. Unless otherwise indicated, a particular nucleic acid sequence also includes degenerate codon substitutions.

Nucleic acids can include one or more regulatory elements operably linked to the open reading frame, where the one or more regulatory elements are configured to direct the transcription and translation of the polypeptide encoded by the open reading frame in a mammalian cell. Non-limiting examples of regulatory elements include transcription initiation sequences (e.g., promoters, enhancers, a TATA box, and the like), translation initiation sequences, mRNA stability sequences, poly A sequences, secretory sequences, and the like. Regulatory elements can be obtained from the genome of any suitable organism. Non-limiting examples of useful regulatory elements include SV40 early promoter, mouse mammary tumor virus LTR promoter; adenovirus major late promoter (Ad MLP); a herpes simplex virus (HSV) promoter, a cytomegalovirus (CMV) promoter such as the CMV immediate early promoter region (CMVIE), a rous sarcoma virus (RSV) promoter, pol II promoters, pol III promoters, synthetic promoters, hybrid promoters, and the like. In addition, sequences derived from non-viral genes, such as the murine metallothionein gene, will also find use herein. Exemplary constitutive promoters include the promoters for the following genes which encode certain constitutive or "housekeeping" functions: hypoxanthine phosphoribosyl transferase (HPRT), dihydrofolate reductase (DHFR), adenosine deaminase, phosphoglycerol kinase (PGK), pyruvate kinase, phosphoglycerol mutase, the actin promoter, and other constitutive promoters known to those of skill in the art. In addition, many viral promoters function constitutively in eukaryotic cells. These include: the early and late promoters of SV40; the long terminal repeats (LTRs) of Moloney Leukemia Virus and other retroviruses; and the thymidine kinase promoter of Herpes Simplex Virus, among many others. Accordingly, any of the above-referenced constitutive promoters can be used to control transcription of a heterologous gene insert.

Genes under control of inducible promoters are expressed only or to a greater degree, in the presence of an inducing agent, (e.g., transcription under control of the metallothionein promoter is greatly increased in presence of certain metal ions). Inducible promoters include responsive elements (REs) which stimulate transcription when their inducing factors are bound. For example, there are REs for serum factors, steroid hormones, retinoic acid and cyclic AMP. Promoters containing a particular RE can be chosen in order to obtain an inducible response and in some cases, the RE itself may be attached to a different promoter, thereby conferring inducibility to the recombinant gene. Thus, by selecting a suitable promoter (constitutive versus inducible; strong versus weak), it is possible to control both the existence and level of expression of a polypeptide in the genetically modified cell. If the gene encoding the polypeptide is under the control of an inducible promoter, delivery of the polypeptide *in situ* is triggered by exposing the genetically modified cell *in situ* to conditions for permitting transcription of the polypeptide, e.g., by intraperitoneal injection of specific inducers of the inducible promoters which control transcription of the agent. For example, *in situ* expression by genetically modified cells of a polypeptide encoded by a gene under the control of the metallothionein promoter, is enhanced by contacting the genetically modified cells with a solution containing the appropriate (i.e., inducing) metal ions *in situ.*

A nucleic acid is "operably linked" when it is placed into a functional relationship with another nucleic acid sequence. A nucleic acid encoding a polypeptide, or a nucleic acid directing expression of a TPP1 polypeptide (e.g., a polypeptide having TPP1 activity) may include an inducible promoter, or a tissue-specific promoter for controlling transcription of the encoded polypeptide.

In certain embodiments, CNS-specific or inducible promoters, enhancers and the like, are employed in the methods described herein. Non-limiting examples of CNS-specific promoters include those isolated from the genes from myelin basic protein (MBP), glial fibrillary acid protein (GFAP), and neuron specific enolase (NSE). Non-limiting examples of inducible promoters include DNA responsive elements for ecdysone, tetracycline, hypoxia and IFN.

A polypeptide can be targeted for delivery to an extracellular, intracellular or membrane location. A gene product secreted from cells typically has a secretion "signal" for secretion from the cell to the extracellular milieu. An expression vector can also be constructed to include a secretion "signal." A gene product may also be retained within the cell. In a similar manner, a gene product may include or the expression vector can be constructed to include "retention" signal sequences for anchoring the polypeptide within the cell plasma membrane. For example, membrane proteins have hydrophobic transmembrane regions, which maintain protein in the membrane.

As used herein, the terms "modify" or "variant" and grammatical variations thereof, mean that a nucleic acid, polypeptide or subsequence thereof deviates from a reference sequence. Modified and variant sequences may therefore have substantially the same, greater or less expression, activity or function than a reference sequence, but at least retain partial activity or function of the reference sequence. A particular type of variant is a mutant protein, which refers to a protein encoded by a gene having a mutation, e.g., a missense or nonsense mutation in TPP1.

A "nucleic acid" or "polynucleotide" variant refers to a modified sequence which has been genetically altered compared to wild-type. The sequence may be genetically modified without altering the encoded protein sequence. Alternatively, the sequence may be genetically modified to encode a variant protein, e.g., a variant TPP1 protein. A nucleic acid or polynucleotide variant can also refer to a combination sequence which has been codon modified to encode a protein that still retains at least partial sequence identity to a reference sequence, such as wild-type protein sequence, and also has been codon-modified to encode a variant protein. For example, some codons of such a nucleic acid variant will be changed without altering the amino acids of a TPP1 protein encoded thereby, and some codons of the nucleic acid variant will be changed which in turn changes the amino acids of a TPP1 protein encoded thereby.

The term "polypeptide" as used herein refers to a polymer of amino acids and includes full-length proteins and fragments thereof. Thus, "protein" and "polypeptide" are often used interchangeably herein. The "polypeptides" encoded by a "nucleic acid" or "polynucleotide" sequence disclosed herein include partial or full-length native TPP1 sequences, as with naturally occurring wild-type and functional polymorphic proteins, functional subsequences (fragments) thereof, and modified forms or sequence variants thereof, so long as the polypeptide retains some degree of TPP1 enzyme activity. Accordingly, in methods as described herein , such polypeptides encoded by nucleic acid sequences can be, but are not required to be, identical to the endogenous protein TPP1 protein that is defective, or whose expression is insufficient, or deficient in a treated mammal.

Amino acid changes in a polypeptide can be achieved by changing the codons of the corresponding nucleic acid sequence. Such polypeptides can be obtained based on substituting certain amino acids for other amino acids in the polypeptide structure in order to modify or improve biological activity. For example, through substitution of alternative amino acids, small conformational changes may be conferred upon a polypeptide that results in increased activity. Alternatively, amino acid substitutions in certain polypeptides may be used to provide residues, which may then be linked to other molecules to provide peptide-molecule conjugates which, retain sufficient properties of the starting polypeptide to be useful for other purposes.

One can use the hydropathic index of amino acids in conferring interactive biological function on a polypeptide, wherein it is found that certain amino acids may be substituted for other amino acids having similar hydropathic indices and still retain a similar biological activity. Alternatively, substitution of like amino acids may be made on the basis of hydrophilicity, particularly where the biological function desired in the polypeptide to be generated in intended for use in immunological embodiments. The greatest local average hydrophilicity of a "protein", as governed by the hydrophilicity of its adjacent amino acids, correlates with its immunogenicity. Accordingly, it is noted that substitutions can be made based on the hydrophilicity assigned to each amino acid.

In using either the hydrophilicity index or hydropathic index, which assigns values to each amino acid, conduct substitutions of amino acids where these values are ±2, with ±1 being typical, and those with in ±0.5 being the most typical substitutions.

Non-limiting examples of modifications include one or more nucleotide or amino acid substitutions (e.g., about 1 to about 3, about 3 to about 5, about 5 to about 10, about 10 to about 15, about 15 to about 20, about 20 to about 25, about 25 to about 30, about 30 to about 40, about 40 to about 50, about 50 to about 100, about 100 to about 150, about 150 to about 200, about 200 to about 250, about 250 to about 500, about 500 to about 750, about 750 to about 1000 or more nucleotides or residues). One non-limiting example of a nucleic acid modification is codon optimization.

An example of an amino acid modification is a conservative amino acid substitution or a deletion. In particular embodiments, a modified or variant sequence (e.g., TPP1) retains at least part of a function or activity of the unmodified sequence (e.g., wild-type TPP1).

A "nucleic acid fragment" is a portion of a given nucleic acid molecule. Deoxyribonucleic acid (DNA) in the majority of organisms is the genetic material while ribonucleic acid (RNA) is involved in the transfer of information contained within DNA into proteins. Fragments and variants of the disclosed nucleotide sequences and proteins or partial-length proteins encoded thereby are also encompassed by the present disclosure. By "fragment" or "portion" is meant a full length or less than full length of the nucleotide sequence encoding, or the amino acid sequence of, a polypeptide or protein. In certain embodiments, the fragment or portion is biologically functional (i.e., retains 5%, 10%, 15%, 20%, 25%, 30%, 35%, 40%, 45%, 50%, 55%, 60%, 65%, 70%, 75%, 80%, 85%, 90%, 95%, 99% or 100% of enzymatic activity of the wild-type TPP1).

A "variant" of a molecule is a sequence that is substantially similar to the sequence of the native molecule. For nucleotide sequences, variants include those sequences that, because of the degeneracy of the genetic code, encode the identical amino acid sequence of the native protein. Naturally occurring allelic variants such as these can be identified with the use of molecular biology techniques, as, for example, with polymerase chain reaction (PCR) and hybridization techniques. Variant nucleotide sequences also include synthetically derived nucleotide sequences, such as those generated, for example, by using site-directed mutagenesis, which encode the native protein, as well as those that encode a polypeptide having amino acid substitutions. Generally, nucleotide sequence variants disclosed herein will have at least 40%, 50%, 60%, to 70%, e.g., 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98%, sequence identity to the native (endogenous) nucleotide sequence. In certain embodiments, the variant is biologically functional (i.e., retains 5%, 10%, 15%, 20%, 25%, 30%, 35%, 40%, 45%, 50%, 55%, 60%, 65%, 70%, 75%, 80%, 85%, 90%, 95%, 99% or 100% of enzymatic activity of the wild type TPP1). According to the claimed invention, the nucleic acid encodes a polypeptide, wherein said polypeptide comprises a TPP1 polypeptide having at least 90% identity to an amino acid sequence set forth in SEQ ID NO:5 or a pro-enzyme thereof.

"Conservatively modified variations" of a particular nucleic acid sequence refers to those nucleic acid sequences that encode identical or essentially identical amino acid sequences. Because of the degeneracy of the genetic code, a large number of functionally identical nucleic acids encode any given polypeptide. For instance, the codons CGT, CGC, CGA, CGG, AGA and AGG all encode the amino acid arginine. Thus, at every position where an arginine is specified by a codon, the codon can be altered to any of the corresponding codons described without altering the encoded protein. Such nucleic acid variations are "silent variations," which are one species of "conservatively modified variations." Every nucleic acid sequence described herein that encodes a polypeptide also describes every possible silent variation, except where otherwise noted. One of skill in the art will recognize that each codon in a nucleic acid (except ATG, which is ordinarily the only codon for methionine) can be modified to yield a functionally identical molecule by standard techniques. Accordingly, each "silent variation" of a nucleic acid that encodes a polypeptide is implicit in each described sequence.

The term "substantial identity" of polynucleotide sequences means that a polynucleotide comprises a sequence that has at least 70%, 71%, 72%, 73%, 74%, 75%, 76%, 77%, 78%, or 79%, or at least 80%, 81%, 82%, 83%, 84%, 85%, 86%, 87%, 88%, or 89%, or at least 90%, 91%, 92%, 93%, or 94%, or even at least 95%, 96%, 97%, 98%, or 99% sequence identity, compared to a reference sequence using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

Substantial identity of amino acid sequences for these purposes normally means sequence identity of at least 70%, at least 80%, 90%, or even at least 95%.

The term "substantial identity" in the context of a peptide indicates that a peptide comprises a sequence with at least 70%, 71%, 72%, 73%, 74%, 75%, 76%, 77%, 78%, or 79%, or 80%, 81%, 82%, 83%, 84%, 85%, 86%, 87%, 88%, or 89%, or at least 90%, 91%, 92%, 93%, or 94%, or even, 95%, 96%, 97%, 98% or 99%, sequence identity to the reference sequence over a specified comparison window. An indication that two peptide sequences are substantially identical is that one peptide is immunologically reactive with antibodies raised against the second peptide. Thus, a peptide is substantially identical to a second peptide, for example, where the two peptides differ only by a conservative substitution. According to the claimed invention, the polypeptide comprises a TPP1 polypeptide having at least 90% identity to an amino acid sequence set forth in SEQ ID NO:5 or a pro-enzyme thereof.

An AAV "genome" refers to a recombinant nucleic acid sequence that is ultimately packaged or encapsulated to form an AAV particle. An AAV particle often comprises an AAV genome. In cases where recombinant plasmids are used to construct or manufacture recombinant vectors, the vector genome does not include the portion of the "plasmid" that does not correspond to the vector genome sequence of the recombinant plasmid. This non vector genome portion of the recombinant plasmid is referred to as the "plasmid backbone," which is important for cloning and amplification of the plasmid, a process that is needed for propagation and recombinant virus production, but is not itself packaged or encapsulated into virus (e.g., AAV) particles. Thus, a vector "genome" refers to nucleic acid that is packaged or encapsulated by virus (e.g., AAV).

A "transgene" is used herein to conveniently refer to a nucleic acid that is intended or has been introduced into a cell or organism. Transgenes include any nucleic acid, such as a gene that encodes a polypeptide or protein (e.g., TPP1).

In a cell having a transgene, the transgene is often introduced/transferred by way of a vector, such as an AAV particle. Introduction of a transgene into a cell by an AAV particle is often referred to as "transduction" of the cell. The term "transduce" refers to introduction of a molecule such as a nucleic acid into a cell or host organism by way of a vector (e.g., an AAV particle). The transgene may or may not be integrated into genomic nucleic acid of a transduced cell. If an introduced nucleic acid becomes integrated into the nucleic acid (genomic DNA) of the recipient cell or organism it can be stably maintained in that cell or organism and further passed on to or inherited by progeny cells or organisms of the recipient cell or organism. Finally, the introduced nucleic acid may exist in the recipient cell or host organism extra chromosomally, or only transiently. A "transduced cell" is a cell into which the transgene has been introduced by way of transduction. Thus, a "transduced" cell is a cell into which, or a progeny thereof in which a nucleic acid has been introduced. A transduced cell can be propagated and the introduced protein expressed, or nucleic acid transcribed. For gene therapy uses and methods, a transduced cell can be in a mammal.

As used herein, the term "serotype" is a distinction used to refer to an AAV having a capsid that is serologically distinct from other AAV serotypes. Serologic distinctiveness is determined on the basis of the lack of cross-reactivity between antibodies to one AAV as compared to another AAV. Such cross-reactivity differences are usually due to differences in capsid protein sequences/antigenic determinants (e.g., due to VP1, VP2, and/or VP3 sequence differences of AAV serotypes). Despite the possibility that AAV variants including capsid variants may not be serologically distinct from a reference AAV or other AAV serotype, they differ by at least one nucleotide or amino acid residue compared to the reference or other AAV serotype.

In various exemplary embodiments, an AAV particle or a vector genome thereof related to a reference serotype has a polynucleotide, polypeptide or subsequence thereof that comprises or consists of a sequence at least 60% or more (e.g., 65%, 70%, 75%, 80%, 85%, 90%, 95%, 96%, 97%, 98%, 99%, 99.1%, 99.2%, 99.3%, 99.4%, 99.5%, etc.) identical to a polynucleotide, polypeptide or subsequence of an AAV1, AAV2, AAV3, AAV4, AAV5, AAV6, AAV7, AAV8, AAV9, AAV10, AAV11, AAV12, Rh10, Rh74 or AAV-2i8 particle. In particular embodiments, an AAV particle or a vector genome thereof related to a reference serotype has a capsid or ITR sequence that comprises or consists of a sequence at least 60% or more (e.g., 65%, 70%, 75%, 80%, 85%, 90%, 95%, 96%, 97%, 98%, 99%, 99.1%, 99.2%, 99.3%, 99.4%, 99.5%, etc.) identical to a capsid or ITR sequence of an AAV1, AAV2, AAV3, AAV4, AAV5, AAV6, AAV7, AAV8, AAV9, AAV10, AAV11, AAV12, Rh10, Rh74 or AAV-2i8 serotype.

The term "about" at used herein refers to a values that is within 10% (plus or minus) of a reference value.

The terms "treat" and "treatment" refer to both therapeutic treatment and prophylactic or preventative measures, wherein the object is to prevent or decrease an undesired physiological change or disorder, such as the development or spread of cancer. For purposes of this disclosure, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The terms "a" and "an" and "the" and similar referents in the context of describing the invention are to be construed to cover both the singular and the plural, unless otherwise indicated herein or clearly contradicted by context. Thus, for example, reference to "a vector" includes a plurality of such vectors, and reference to "a virus" or "particle" includes a plurality of such virions/particles.

The terms "comprising," "having," "including," and "containing" are to be construed as openended terms (i.e., meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described herein.

All of the features disclosed herein may be combined in any combination. Each feature disclosed in the specification may be replaced by an alternative feature serving a same, equivalent, or similar purpose. Thus, unless expressly stated otherwise, disclosed features are an example of a genus of equivalent or similar features.

All numerical values or numerical ranges include integers within such ranges and fractions of the values or the integers within ranges unless the context clearly indicates otherwise. Thus, to illustrate, reference to 80% or more identity, includes 81%, 82%, 83%, 84%, 85%, 86%, 87%, 88%, 89%, 90%, 91%, 92%, 93%, 94% etc., as well as 81.1%, 81.2%, 81.3%, 81.4%, 81.5%, etc., 82.1%, 82.2%, 82.3%, 82.4%, 82.5%, etc., and so forth.

Reference to an integer with more (greater) or less than includes any number greater or less than the reference number, respectively. Thus, for example, a reference to less than 100, includes 99, 98, 97, etc. all the way down to the number one (1); and less than 10, includes 9, 8, 7, etc. all the way down to the number one (1).

As used herein, all numerical values or ranges include fractions of the values and integers within such ranges and fractions of the integers within such ranges unless the context clearly indicates otherwise. Thus, to illustrate, reference to a numerical range, such as 1-10 includes 1, 2, 3, 4, 5, 6, 7, 8, 9, 10, as well as 1.1, 1.2, 1.3, 1.4, 1.5, etc., and so forth. Reference to a range of 1-50 therefore includes 1, 2, 3, 4, 5, 6, 7, 8, 9, 10, 11, 12, 13, 14, 15, 16, 17, 18, 19, 20, etc., up to and including 50, as well as 1.1, 1.2, 1.3, 1.4, 1.5, etc., 2.1, 2.2, 2.3, 2.4, 2.5, etc., and so forth.

Reference to a series of ranges includes ranges which combine the values of the boundaries of different ranges within the series. Thus, to illustrate reference to a series of ranges, for example, of 1-10, 10-20, 20-30, 30-40, 40-50, 50-60, 60-75, 75-100, 100-150, 150-200, 200-250, 250-300, 300-400, 400-500, 500-750, 750-1,000, 1,000-1,500, 1,500-2,000, 2,000-2,500, 2,500-3,000, 3,000-3,500, 3,500-4,000, 4,000-4,500, 4,500-5,000, 5,500-6,000, 6,000-7,000, 7,000-8,000, or 8,000-9,000, includes ranges of 10-50, 50-100, 100-1,000, 1,000-3,000, 2,000-4,000, etc.

Embodiments of the invention are described herein. Variations of those embodiments falling within the scope of the invention may become apparent to those of ordinary skill in the art upon reading the foregoing description.

The invention will now be illustrated by the following non-limiting Example.

### Example 1: AAV-Mediated Transduction of Ventricular Lining Cells Delays

### Neurodegenerative Disease Onset and Progression in a Canine Model of the Late Infantile Form of Batten Disease.

The therapeutic effect of CLN2 gene delivery via an AAV vector to the epithelia lining of the brain ventricular system (e.g., ependyma) was evaluated in a LINCL dog model. LINCL dogs are engineered with a defect in the CLN2 gene resulting in a TPP1 enzyme deficiency. The LINCL dogs are normal at birth, but develop neurological signs around 7 months, testable cognitive deficits at ~ 5-6 months, seizures at 10-11 months, and progressive visual loss. The CLN2 gene mutation in the LINCL dog renders the TPP1 protein non-functional, and TPP1 protein is undetectable. With disease progression, brain tissues shrink, leading to enlarged ventricular spaces in the brain. Neurological symptoms include decline in balance and motor functions, loss of vision, tremors. Prior to the instant invention, the effectiveness of ependymal cell transduction with rAAVs expressing lysosomal hydrolases to reverse phenotypes in large animal models was not known.

The transduction of ependymal cells, which have direct access to the CSF, were studied to see if it would provide for long term and wide spread biodistribution of TPP1 in the LINCL dog model following a single unilateral infusion of rAAV expressing the canine form of TPP1 (caTPP1). The ependyma compose the single-layer epithelia lining the brain ventricular system and spinal cord central canal. Ependymal cells are multiciliated and post-mitotic and they are essential for directional CSF flow and movement of paracrine signals, metabolites and toxins through and out of the brain. More specifically, ependymal cells were transduced with rAAV2 expressing canine TPP1 (rAAV.caTPP1) and TPP1 enzyme activity through the brain and outside of CNS evaluated along with clinical benefit.

A one-time administration of recombinant adeno-associated virus (rAAV) expressing TPP1 mediated gene transfer to ependymal cells provided constitutive, high level expression of recombinant TPP1 in the CSF, resulting in remarkable clinical benefit. In particular, rAAVca.TPP1 treated diseased dogs exhibited (1) delays in onset of clinical signs; (2) delays in disease progression; (3) protection from cognitive decline; and (4) extension of lifespan. By immuno-staining and enzyme assay, recombinant protein was evident throughout the brain. In addition, there was correction of neuropathology characteristic of the disease. These studies in the naturally occurring canine disease model indicates the viability of targeting ventricular lining cells to accomplish stable secretion of recombinant protein for broad CNS distribution and therapeutic benefit.

### Materials and Methods.

**AAV Vector Production:** Recombinant AAV2 vectors expressing canine TPP1 under control of the CMV early enhancer/chicken β-actin (CAG) promoter were generated using standard triple transfection methods and purification by CsCl gradient centrifugation (Wright, 2008; Wright, 2009). Titers were quantified by silver stain after gel electrophoresis (SDS-PAGE) and PCR (Wright, 2008; Wright, 2009).

CAG Promoter (SEQ ID NO:9):

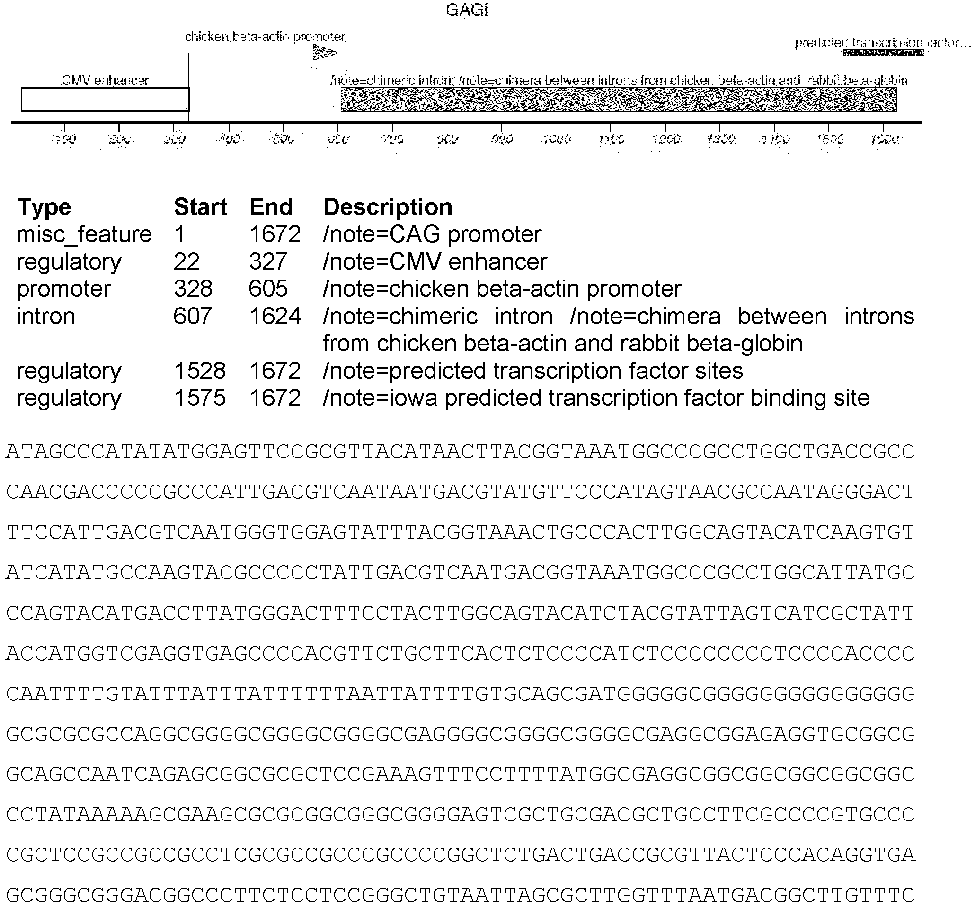
GAGi


chicken beta-actin promoter

predicted transcription factor...

CMV enhancer

/note=chimeric intron; /note=chimera between introns from chicken beta-actin and rabbit beta-globin

100 200 300 400 500 600 700 800 900 1000 1100 1200 1300 1400 1500 1600

Type | Start | End | Description
misc_feature | 1 | 1672 | /note=CAG promoter
regulatory | 22 | 327 | /note=CMV enhancer
promoter | 328 | 605 | /note=chicken beta-actin promoter
intron | 607 | 1624 | /note=chimeric intron /note=chimera between introns from chicken beta-actin and rabbit beta-globin
regulatory | 1528 | 1672 | /note=predicted transcription factor sites
regulatory | 1575 | 1672 | /note=iowa predicted transcription factor binding site

ATAGCCCATATATGGAGTTCCGCGTTACATAACTTACGGTAAATGGCCCGCCTGGCTGACCGCC

CAACGACCCCCGCCCATTGACGTCAATAATGACGTATGTTCCCATAGTAACGCCAATAGGGACT

TTCCATTGACGTCAATGGGTGGAGTATTTACGGTAAACTGCCCACTTGGCAGTACATCAAGTGT

ATCATATGCCAAGTACGCCCCCTATTGACGTCAATGACGGTAAATGGCCCGCCTGGCATTATGC

CCAGTACATGACCTTATGGGACTTTCCTACTTGGCAGTACATCTACGTATTAGTCATCGCTATT

ACCATGGTCGAGGTGAGCCCCACGTTCTGCTTCACTCTCCCCATCTCCCCCCCCTCCCCACCCC

CAATTTTGTATTTATTTATTTTTTAATTATTTTGTGCAGCGATGGGGGCGGGGGGGGGGGGGGG

GCGCGCGCCAGGCGGGGCGGGGCGGGGCGAGGGGCGGGGCGGGGCGAGGCGGAGAGGTGCGGCG

GCAGCCAATCAGAGCGGCGCGCTCCGAAAGTTTCCTTTTATGGCGAGGCGGCGGCGGCGGCGGC

CCTATAAAAAGCGAAGCGCGCGGCGGGCGGGAGTCGCTGCGACGCTGCCTTCGCCCCGTGCCC

CGCTCCGCCGCCGCCTCGCGCCGCCCGCCCCGGCTCTGACTGACCGCGTTACTCCCACAGGTGA

GCGGGCGGGACGGCCCTTCTCCTCCGGGCTGTAATTAGCGCTTGGTTTAATGACGGCTTGTTTC

TTTTCTGTGGCTGCGTGAAAGCCTTGAGGGGCTCCGGGAGGGCCCTTTGTGCGGGGGGAGCGGC
TCGGGGGGTGCGTGCGTGTGTGTGTGCGTGGGGAGCGCCGCGTGCGGCTCCGCGCTGCCCGGCG
GCTGTGAGCGCTGCGGGCGCGGCGCGGGGCTTTGTGCGCTCCGCAGTGTGCGCGAGGGGAGCGC
GGCCGGGGGCGGTGCCCCGCGGTGCGGGGGGGCTGCGAGGGGAACAAAGGCTGCGTGCGGGGT
GTGTGCGTGGGGGGGTGAGCAGGGGGTGTGGGCGCGTCGGTCGGGCTGCAACCCCCCCTGCACC
CCCCTCCCCGAGTTGCTGAGCACGGCCCGGCTTCGGGTGCGGGGCTCCGTACGGGGCGTGGCGC
GGGGCTCGCCGTGCCGGGCGGGGGGTGGCGGCAGGTGGGGGTGCCGGGCGGGGCGGGGCCGCCT
CGGGCCGGGGAGGGCTCGGGGGAGGGGCGCGGCGGCCCCCGGAGCGCCGGCGGCTGTCGAGGCG
CGGCGAGCCGCAGCCATTGCCTTTTATGGTAATCGTGCGAGAGGGCGCAGGGACTTCCTTTGTC
CCAAATCTGTGCGGAGCCGAAATCTGGGAGGCGCCGCCGCACCCCCTCTAGCGGGCGCGGGGCG
AAGCGGTGCGGCGCCGGCAGGAAGGAAATGGGCGGGGAGGGCCTTCGTGCGTCGCCGCGCCGCC
GTCCCCTTCTCCCTCTCCAGCCTCGGGGCTGTCCGCGGGGGGACGGCTGCCTTCGGGGGGGACG
GGGCAGGGCGGGGTTCGGCTTCTGGCGTGTGACCGGCGGCTCTAGAGCCTCTGCTAACCATGTT
CATGCCTTCTTCTTTTTCCTACAGCTCCTGGGCAACGTGCTGGTTATTGTGCTGTCTCATCATT
TTGGCAAA

**Animals:** Long-haired miniature Dachshunds were bred and housed at the University of Missouri in facilities overseen by the Office of Animal Resources (OAR). TPP1-null animals were generated from heterozygous breeding (Awano, 2006). All puppies were genotyped by PCR. In addition to the standard care provided by the OAR, the dogs were socialized on a daily basis throughout the study. All procedures involving dogs were reviewed and approved by the University of Missouri Animal Care and Use Committee and conformed to the National Institutes of Health Guide for the Care and Use of Laboratory Animals. Intraventricular infusions were performed over 25 minutes using the Brainsight^{™} neuronavigation system equipped with a 26 gauge 12 cm needle. Cyclosporine 10 mg/kg twice daily by mouth was administered to all of the dogs in this study starting one to two weeks prior to the gene therapy vector administration. The dose was gradually tapered after 2 months to once daily, which was continued for the duration of the study. A 10-20 mg/kg daily dose by mouth of mycophenolate mofetil (MMF) was instituted, as indicated in the results, and tapered after 2 to several months depending on tolerability.

**Tissue Samples TPP1 enzyme activity assay:** TPP1 activity was assayed via a modification of previously described methods (Chang et al., 2008; Tian et al., 2006). Samples were homogenized in 300 µl ice-cold homogenization buffer (0.1% Triton X-100 in normal saline with complete protease inhibitor cocktail, Roche, Mannheim, Germany). Insoluble material was removed by centrifugation at 21×10³ rcf at 4°C × 15 minutes, and protein quantified by DC Protein Assay (Biorad, Hercules, CA). Supernatant (10 µl) was placed into 96-well black wall plate wells containing 90 µl of 100 mM sodium citrate buffer, 150 mM NaCl, and 0.1% Triton X-100 (pH 4.0) and enzyme substrate (250 µmol/l Ala-Ala-Phe 7-amido-4-methylcoumarin in sodium citrate buffer, pH 4.0). Plates were read in a Safire 2 microplate reader (Tecan., Mannedorf, Switzerland) at 37°C using the 460-nm emission filter. Purified recombinant TPP1 was used as standard (P. Lobel, State University of New Jersey).

**Cerebrospinal fluid TPP1 enzyme activity assay:** Samples were concentrated 3.5-fold using Amicon Ultra-0.5 Centrifugal filter (Ultracel-10K. Millipore Corporation) at 21 × 10³ rcf for 5 minutes at 4°C. Activated TPP1 was quantified as in tissue samples.

**caTPP1 neutralization antibody assay:** To assess neutralizing Abs (Nabs) in CSF, Cln2-/- mouse embryo fibroblast cells were used. Sixteen hours before the assay, 1.0×10⁵ cells/well were plated onto 24 wells plate the day before, and dog CSF was heat inactivated at 56°C for 30 minutes, diluted 1:10, and then pre-incubated with 1.75 nM of TPP1 at 37°C for one hour. The mixture was applied to cells for three hours at 37°C after which the cells were washed with 37°C HBSS (Ca²⁺ and Mg ²⁺) and then homogenized in 100 µl of ice cold homogenization buffer-0.1% TritonX-100 in normal saline with complete protease inhibitor cocktail (Roche, Mannheim, Germany). Insoluble material was removed by centrifugation at 21×10³ rcf at 4°C for 10 minutes and TPP1 activity detected as described above. The results were calculated as percentage of activity obtained from CSF collected prior to vector administration.

**Brain tissue process and histology:** Brain sections obtained shortly after euthanasia were fixed in 4% paraformaldehyde for 24 hours at 4°C. Sections (40 µm) were cut on a freezing microtome and floated in cryoprotectant solution (30% ethylene glycol, 15% sucrose, 0.05% sodium azide, in Tris buffered saline) for storage at -20 °C. Immunohistochemistry was done using: mouse anti-TPP1 (Abcam; 1:500) mouse anti-GFAP (Sigma-Aldrich, 1:500), rabbit anti-SCMAS (generous gift from E.F. Neufeld., University of California, Los Angeles, CA.; 1:500). All antibodies were diluted in Tris-buffered saline with 2% bovine serum albumin, 0.1% NaN₃, and 0.05% Tween 20. Secondary antibody used was biotinylated goat anti-mouse (Jackson, Immuno Research, West Grove, PA; 1:200). Slides were incubated with Vectstain avidin and biotinylated horseradish peroxidase macromolecular complex (ABC) solution (Vector Laboratories) and then developed with 3,3'-diaminobenzidine peroxidase substrate (Sigma-Aldrich). Sections were visualized and photographed using a DM6000B Leica microscope equipped with a DFC450 Leica color camera.

**Western Blot Analysis:** Protein concentration was determined by BCA assay (Pierce, Rockford) and 50 µg of sample applied to 12% SDS-PAGE and then transferred to 0.2 µm Immobilon PVDF membranes (Millipore, Billerica). Primary antibodies were: rabbit anti-p62 (Cell Signaling; 1:1000), rabbit anti-SCMAS (generous gift from E.F. Neufeld; 1:3000). Secondary antibodies were HRP-goat anti-rabbit IgG (Jackson Laboratories; 1:40000). Blots were developed using ECL Plus Western Blotting Detection System (GE Healthcare, Pittsburg). Protein quantification was performed using the VersaDoc^{™} Imaging System (Biorad). Band densities were normalized to total protein loads assessed by Ponceau S staining.

**Brain autofluorescence:** Autofluorescent storage material was assessed on 40 µm free-floating sections mounted onto glass slides cover-slipped with Vectashield. Slides were viewed and photographed under epifluorescence with a DM6000B Leica microscope equipped with an apochromat ×40 magnification lens (N.A. 0.85), excitation bandpass 470(+20): emission bandpass 525(+25) nm filter, and a OrcaFlash 4.0 monochrome camera (Hamamatsu) Exposure-matched digital images were taken from comparable regions of treated and untreated brains. Images were pseudocolored to green as filter emission wavelength.

**Statistical Analyses:** GraphPad Prism 6 software (GraphPad Software, La Jolla California, USA) was used for statistical analyses. Data was analyzed by nonparametric tests. Mann-Whitney test was performed when only two groups were compared or Kruskal-Wallis tests in the case of three or more groups.

**Cognitive testing:** Cognitive testing was performed by assessing the dogs' performance on a reversal learning task as described previously (Sanders et al., 2011). Dogs each began a pre-training phase of the testing protocol and then their performance on the reversal learning task was performed at monthly intervals starting at 4 months of age. Testing continued until the dogs were unable to complete the test due to disease-related disabilities. Data were recorded as the number of errors each dog made during a testing session prior to reaching a predetermined performance criterion.

**Neurologic Examination Summary:** Dogs underwent clinical assessments that encompassed physical and neurologic examinations on a weekly basis. Body weights were recorded in conjunction with physical examinations on all dogs prior to the first dose to establish baseline values and were recorded weekly thereafter. A neurologic examination was performed weekly throughout the course of the study. Signs of neurologic dysfunction were subjectively monitored by a standardized clinical neurologic examination (Lorenz et al., 2011). Components of the neurologic examination included observation of mentation, posture, and gait; testing of cranial nerves; evaluation of postural reactions (proprioceptive placement, hopping, wheelbarrow, tactile placement, and extensor postural thrust); spinal reflexes (myotatic and flexor); and sensory testing. Gait evaluation was assessed as normal or abnormal with presence of ataxia (general proprioceptive, cerebellar, vestibular) and para- and tetraparesis. Postural reactions, spinal reflexes, cranial nerve tests, and sensation were assessed as intact, decreased, or absent. Dogs were also evaluated for abnormal movement and seizure activities. Age at onset was recorded for the following neurologic deficits: menace response deficits (unilateral and bilateral), visual tracking, intention tremor, head tremor, myoclonic jerks, ataxia, and proprioceptive placement of pelvic and thoracic limbs.

For humane reasons, euthanasia was performed by using a uniform criterion for defining end-stage disease. End-stage disease criteria consisted of loss of cognition, severe mentation abnormalities, loss of visual tracking, medication refractory myoclonic jerk and seizure activity, and inability to eat without significant assistance. Survival time was defined as the time from birth to sacrifice regardless of the clinical signs that led to decisions to euthanize.

### RESULTS.

**TPP1 Expression in canine CSF:** The TPP1-null Dachshund disease model has a frameshift mutation in CLN2 (Awano et al., 2006) with no detectable TPP1 activity in blood, and progressive neurodegenerative symptomatology that recapitulates human TPP deficiency (Sanders et al., 2011; Katz et al. 2008).

To determine if ependymal transduction is sufficient for widespread delivery of recombinant TPP1 expression in brain required a vector that, when delivered, would infect the ependyma specifically and with reasonable efficiency. In mice, AAV4 is unique in that intrastriatal or intraventricular injection results in robust ependyma transduction (Davidson et al., 2000). In contrast, no ependyma transduction was observed in canine brain after AAV4 delivery. AAV1, 2, 6, 8 and 9 serotypes expressing reporter genes were additionally screened and AAV2 was found to be optimal; intraventricular injection of 2E12 vector genomes resulted in nearly complete transduction of ependyma lining the lateral ventricles, and additional transduction of ependyma in the 3^{rd} and 4^{th} ventricles.

TPP1 enzyme activity levels early after unilateral injection of rAAV. caTPP 1 (DC013, DC014) into the CSF in two CLN2-/- dogs was quantified (Table 1; all dogs started on cyclosporine prior to treatment).

**Table 1: Drug and Vector Treatments**

| Dog | AAV2/2 dose | AAV titer | MMF Treatment (days from vector injection) |
|---|---|---|---|
| DC013 | 1500 µl | 1.1 × 10¹³ vg/ml | 44 d |
| DC014 | 1500 µl + 200 µl | 1.1 × 10¹³ vg/ml | 44 d |
| DC015 | 1500 µl + 1500 µl | 1.1 × 10¹³ vg/ml | 33 d |
| DC016 | 1500 µl + 1500 µl | 1.1 × 10¹³ vg/ml | 33 d |
| DC018 | 1500 µl | 1.1 × 10¹³ vg/ml | -5 d |
| DC019 | 1500 µl | 1.1 × 10¹³ vg/ml | -5 d |
| DC020 | 1500 µl | 1.1 × 10¹³ vg/ml | -5 d |
| DC024 | - | - | -5 d |
| DC025 | - | - | - |

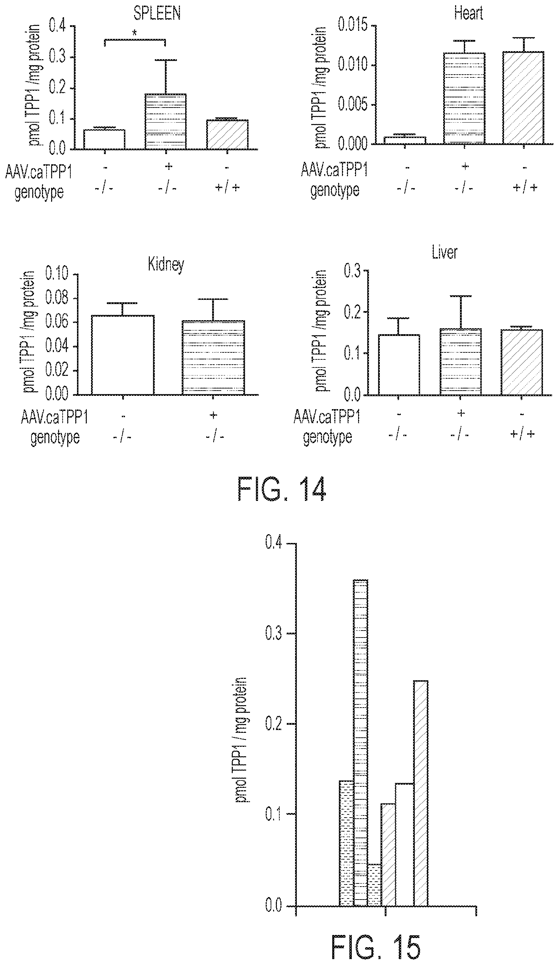

By as early as 5 days post-injection there were significant increases in TTP1 enzyme activity in the CSF; up to 20-fold greater than levels measured in heterozygous control dog CSF (FIG.7A; FIG. 15). However, recombinant TPP1 activity subsided to background levels two months later (FIG.7A). As these dogs are TPP1 null, it was tested if this decline was coincident with the elevation of neutralizing antibodies (NAb). Purified TTP1 proenzyme was incubated with serial dilutions of CSF or serum collected from r AA V. ca TPP 1 treated CLN2-/- dogs, and then the mixture applied to Cln2-/- mouse embryonic fibroblasts. It was found that TPP1 activity in Cln2-/- cell lysates was reduced when the recombinant protein was incubated with CSF of rAAV.caTPP1 treated dogs collected one-month post-injection (FIG.7B). Sera from these animals showed similar results.

In an attempt to suppress neutralizing immune responses, MMF treatment was instituted in addition to ongoing cyclosporine treatment. Two dogs (DC013 and DC014) were administered MMF starting at 44 days, and another 2 at 33 days (DC015 and DC016). The introduction of MMF 44 days post-vector administration did not result in a recovery of TPP1 activity (FIG.7A). However, initiating MMF treatment by 33 days post-vector administration resulted in an increase in CSF TPP1 activity and a reduction in anti-TPP1 Ab titers (FIGS.7C and 7D). Next, a CLN2-/- dog was treated with MMF 5 days prior to rAAV.caTPP1 delivery (DC018). In this animal, TPP1 activity was robust and sustained, and anti-TPP1 antibodies did not appear (FIGS.7E and 7F). Given the stability of transgene expression and overall improvements in neurological symptoms an additional 2 dogs (DC019 and DC020) were treated with rAAV.caTPP1 at day 5 following initiation of MMF therapy.

### Elevated CSF levels of TPP1 are associated with improved clinical symptoms and cognitive deficits.

Untreated CLN2-/- dogs show onset of clinical signs as early as 5 months of age and generally require humane sacrifice by 38-45 weeks (Katz et al., 2014). One of the earliest clinical signs in untreated CLN2-/- dogs is an impaired proprioceptive response in the hind limbs (mean age at onset=6 mo.; range 4.6-8 mo.; n=8; FIG.8A) and hindlimb weakness. These clinical phenotypes were delayed 4.5 months on average, with rAAV.caTPP1 gene therapy (FIG.8A). In three dogs, abnormal nystagmus never appeared (mean age onset=7.7 mo. in untreated dogs, range 4.6-10.9, n=7; treated dogs 13.4 mo., range 12.6-14.3, n=2) (FIG.8B), nor did menace response deficits (mean age at onset in untreated dogs=6.5 mo., range 4.8-8,3, n=6; treated dogs 10 mo., range 9.9-10.2, n=2) (FIG.8C). Pupillary light reflex (PLR) abnormalities were delayed in 3, and never detected in 2, treated dogs (onset is at mean age of 8.5 months in untreated dogs, range 6.7-9.6 months, n=8; treated dogs 11.6 mo., range 10.6-13.7; n=3) (FIG.8D). Cerebellar ataxia, which typically has onset shortly after hind-limb proprioceptive deficits, was delayed from a mean age of 6.4 months in untreated dogs (range 5.9-9.4months, n=8) to 12.7 months in treated dogs (range 12-13.8 months, n=4) and never appeared in 1 of the treated animals (FIG.8E). Thoracic limb dysfunction was also delayed by 5 to 5.5 months, and never appeared in 1 treated animal (FIG.8F).

Onset of neuroanatomic localized abnormalities varied between treated dogs compared to untreated dogs. The earliest abnormalities in untreated dogs is general proprioceptive ataxia, but were visual deficits in the treated dogs. This likely reflects a lack of retinal penetration of recombinant protein secreted into the CSF. Nonetheless, there was notable sparing of the central pathways involved in the PLR, which are initiated by the retina (FIGS.8C and 8D). Overall, there were significant delays in the onset of movement abnormalities, the most striking example being the delay in onset or absence of intention tremor (FIG.8G), abnormal nystagmus and cerebellar ataxia indicating sparing of the cerebellovestibular system.

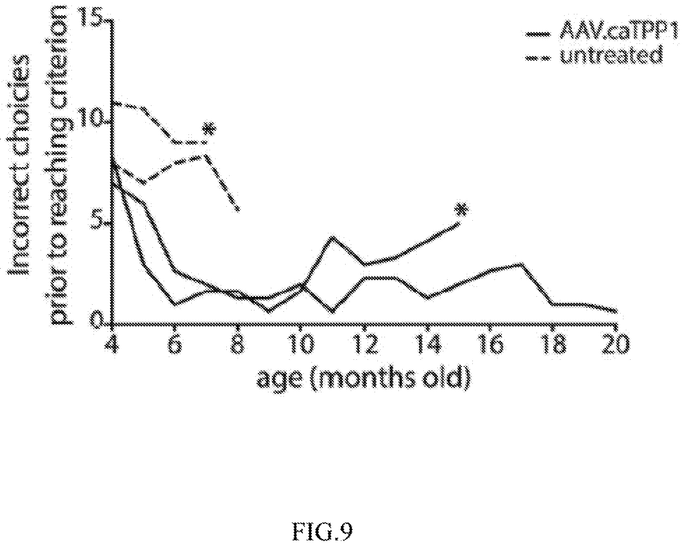

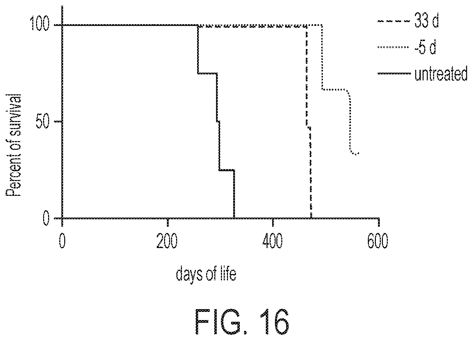

In addition to neurological signs, CLN2-/- dogs have cognitive decline that can be quantified by a Tmaze (Sanders et al., 2011). Four animals were enrolled, 2 treated and 2 untreated, in this assay beginning at 4 months of age. Similar to earlier work (Sanders et al., 2011), untreated CLN2-/- animals made more mistakes on the T-maze and became unable to complete the task by 6 months of age (FIG.9). However, dogs receiving rAA V.caTPP1 continued to navigate the maze similar to normal dogs as long as their motor abilities allowed (FIG.9). One treated animal continues to perform similar to normal dogs with very few mistakes at 19 months of age, and remains highly interactive with his environment, caregivers and other dogs in the colony. The extension of lifespan (FIG.16; mean age to humane sacrifice is 10.4 months in untreated dogs compared to 16.4 months in rAAV2.caTPP1 treated dogs (dynamic; one dog remains alive past 22 months; Table 1), delay in clinical symptoms (FIG.8), and retention of cognitive abilities (FIG.9) is remarkably extended from the improvements noted with biweekly infusion of recombinant TPP1 into the intrathecal or ventricular space of dogs from the same colony (Katz et al., 2014).

### Recombinant TPP1 is widely distributed in brain parenchyma.

Thus far, the data support the hypothesis that rAAV.caTPP1 gene transfer to ependyma provides TPP1 activity in brain sufficient for clinical benefit. It is expected that recombinant TPP1 in the CSF flows from the ventricles into the subarachnoid space via the lateral apertures and ultimately diffuses along perivascular spaces to the underlying parenchyma. In rodents, this results in improvement in disease-associated readouts (Chang et al., 2008; Liu et al., 2005). However, this earlier work was done in rodents and it was not known if, in a larger brain, there would be widespread delivery, regional variability in TPP1 penetration or if uptake would be for certain cell types.

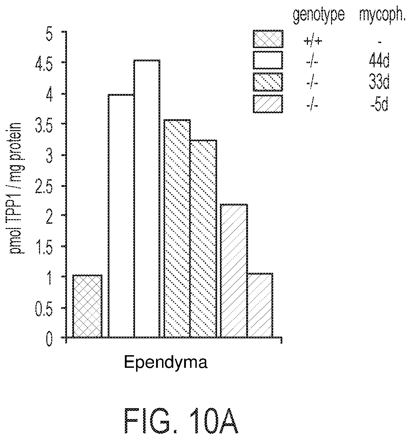

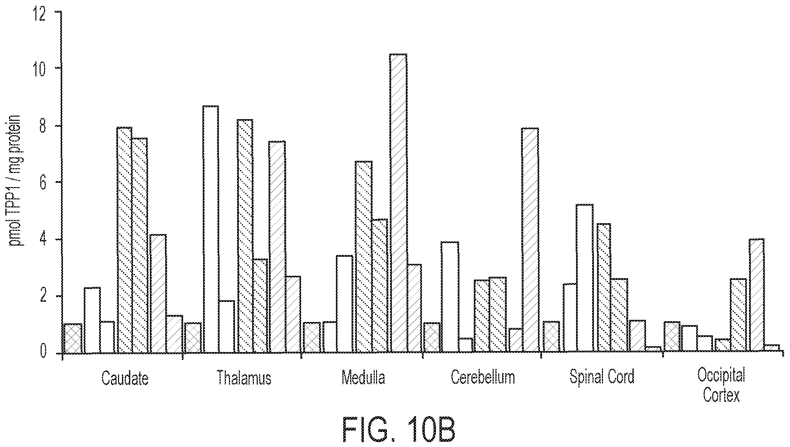

Significant increases were found in TPP1 activity from rostral to caudal brain regions in tissues harvested 60 days (2 months; DC013 and DC014), 300 days (10 months; DC015 and DC016), 444 days (~16 months; DC018), and 545 days (~19 months; DC017) post-injection. DC019 remained cognitively intact (~22 months; 631 days). TPP1 activity was increased 2- and 4.5-fold over normal in areas proximal to the lateral ventricle (FIG. 10A). More than 8-fold over normal was observed in samples harvested from the caudate nucleus, thalamus and medulla (FIG. 10B). TPP1 activity was also detected in the occipital cortex, demonstrating that the can reach the subarachnoid space and penetrate the cerebral cortex (FIG.10B).

To further examine TPP1 distribution in brain, immunohistochemistry for canine TPP1 was performed. Robust enzyme staining was found throughout the brain, in contrast to null animals (FIG. 11). TPP1 was evident in ependymal cells lining the entire ventricular system as well as in regions surrounding the lateral ventricle (e.g., septum and caudate; FIG.11A), the 3^{rd} ventricle (e.g., hypothalamus; FIG.11A), the 4^{th} ventricle (e.g., the rostral medulla; FIG.11A), and central canal of the spinal cord (FIG. 11B). TPP1-positive cells were also found along the cerebral and cerebellar cortices of rAAV.caTPP1 injected animals (FIG.11C). A rostral-caudal gradient of immune-positive cells was found. TPP1-positive cells were morphologically equivalent to pyramidal neurons, with punctate immune-reactivity within the soma and the major dendrites. In the prefrontal cortex, most TPP1-positive cells were in layer V (FIG.11C). More caudally, TPP1 immune positive cells were found in all cortical layers (FIG. 11C). In the piriform cortex the intracellular immunoreactivity was present in the soma and multiple cellular processes (FIG.11C). In the cerebellar cortex, the Purkinje neurons and other cells within the molecular layers showed the most robust TPP1 staining (FIG.11C). Together these results corroborate that enzyme secreted from ependyma can reach the subarachnoid space, and be internalized through perivascular spaces throughout multiple brain regions.

### Decreased astrocyte activation and storage burden after rAAV2.caTPP1 transduction.

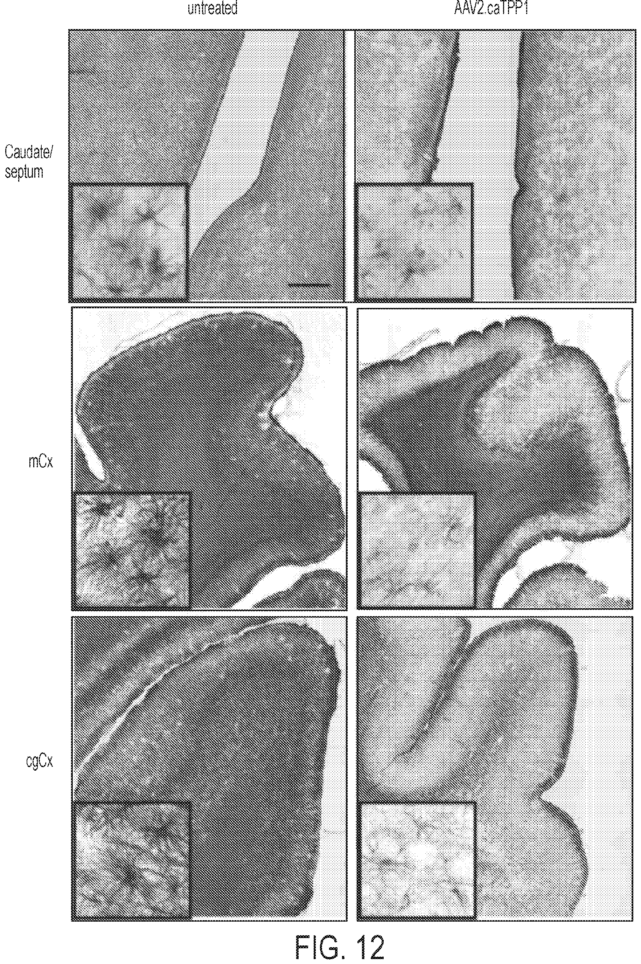

Progressive astrocyte activation has been described in rodent models of TPP-1 deficiency, and in patient brain. This activation is characterized by increased intensity of GFAP immunostaining in astrocytic processes. Intense GFAP staining was found in untreated TPP1-null dogs (FIG.12). Ependymal cell transduction with rAAV.caTPP1 resulted in a notable decrease in GFAP immunoreactivity even at end stage disease (FIG.12). The septum, caudate nucleus, motor cortex (mCx) and cingulate cortex (cgCx) in AAV.caTPP1 injected animals showed weak GFAP staining by IHC, and fewer immunoreactive processes relative to those areas in untreated TPP1-deficient dogs (FIG. 12). Cumulatively the data indicate that the presence of TPP1 in these animals reduced the progressive astrocytosis.

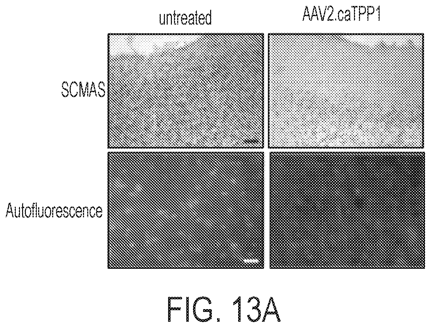

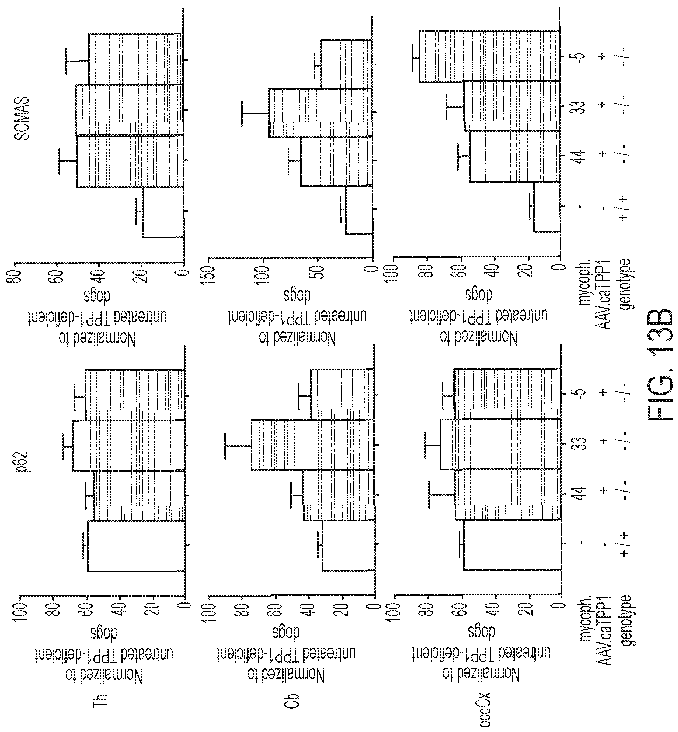

Autofluorescent accumulations are a hallmark of the NCLs, and are evident throughout disease brain. AAV2.caTPP1 significantly reduced autofluorescent storage in X, Y and Z regions (FIG.13A). This was accompanied by a measurable decrease in ATP synthase subunit C accumulation (SCMAS) evaluated by immunohistochemistry (FIG.13A) and western blot (FIG.13B). p62 levels were also evaluated, which were recently reported to be elevated in TPP1 null mice (Micsenyi et al., 2013). AAV2.caTPP1 significantly reduced p62 storage burden relative to that found in untreated animals (FIG. 13B).

### Recombinant TPP1 in peripheral organs.

Although the most relevant symptoms in children with mutations in CLN2 are neurological in origin, TPP1 is a ubiquitously expressed enzyme (Kida et al., 2001). TPP1 expressed in CSF after ependymal transduction may provide a source of enzyme outside CNS, as protein in CSF can pass through the arachnoid villi to reach the peripheral vasculature. To determine distribution outside of CNS in treated dogs, recombinant TPP1 was quantified by enzymatic activity assay in liver, spleen, kidney and heart in untreated and AA V.caTPP1-treated dogs. Significantly elevated TPP1 activity was found in spleen and heart in AAV.caTPP1 treated dogs relative to controls (FIG.14). While total activity levels are elevated, immunohistochemistry of available heart samples showed patchy, robust staining in some myoblasts, with more uniform staining in spleen. There was no significant increase in kidney, and TPP1 activity in the liver was also not significantly elevated due to enhanced background enzyme activity in null, untreated dogs, likely due to another hydrolase in liver capable of cleaving the substrate used in this assay (FIG. 15). Normalization of TPP1 activity in heart is relevant to the clinical manifestation in children, as cardiomyopathies have been reported and may contribute to disease progression in another of the NCLs (Ostergaard et al., 2011). Enzymatic activity in AAV.caTPP1 treated dogs was an order of magnitude greater than in untreated null animals in heart, and 2-fold higher in spleen (FIG. 14). These results support that a CSF-resident enzyme can provide a source of TPP1 for peripheral organs following ependymal transduction.

### DISCUSSION

Safety studies of gene therapy for LINCL due to TPP1 deficiency have been reported in patients, employing 12 injections of recombinant AAV2 into the brain parenchyma (Worgall et al., 2008). A second clinical study using similar delivery methods has been initiated with AAV serotype rh10 delivered via intra-parenchymal injection (ClinicalTrials.gov Identifier: NCT01161576). In both trials, virus is restricted to the cerebral parenchyma.

To explore if recombinant protein delivery to brain and perhaps a broader distribution could be achieved via the CSF, ependymal transduction following a single intraventricular injection, or a combined intraventricular and cisternal injection was evaluated. Moreover, in contrast to parenchyma, these methods do not require major neurosurgery requiring extended sedation, an important consideration for children with a neurodegenerative disease. In dogs, this approach resulted in broad distribution of recombinant TPP1, with significant delays in disease onset, disease progression, and protection from cognitive decline. This therapy also produced neurological improvements and increased life span, and improved quality of life.

A striking observation was the difference in the order in which clinical signs first appeared. Untreated TPP1-deficient dogs first display deficits in proprioceptive placement and ataxia with weakness of the hind limbs. They also show very early cognitive impairment reflected by poor performance in the T-maze. The earliest symptoms in children with TPP1 deficiency are similar. Affected children generally display early language deficits and either a delay in developmental milestones or a loss of milestones already acquired, as well as ataxia and proprioceptive deficits. In AAV2.caTPP1 treated animals, the features of ataxia, proprioceptive deficits, and cognition were dramatically delayed as regards onset, and in some treated animals ataxia, proprioceptive deficits, and loss of cognition never appeared. Instead, the earliest clinical signs in AAV2.caTPP1 treated animals were visual abnormalities. Nonetheless, treated dogs showed delay of clinical signs that would be caused by defects in central pathways receiving retinal input, and histological and enzyme assay results show extensive levels of TPP1 present in these regions and other regions necessary for normal vision. Thus, directed correction of the retina via intraocular injection could likely preserve vision in these subjects.

Histologically, reduction in storage burden and glial activation in many areas were detected, but the results were variable as to the extent of that reduction. TPP1-deficient dogs with the most profound clinical benefit had lower levels of residual storage. Untreated dogs, sacrificed from 9-11 months of age, had extensive storage and glial activation throughout all brain regions, similar to TPP1 null mice (Sleat et al., 2004). Tissues from treated animals harvested at 13-17 months of age (10-14 months after gene transfer) showed extensive reductions in SCMAS, autofluorescence inclusions, p62 levels and glial activation. These data are interesting in light of the fact that in some animals there were obvious anti-TPP1 responses to the recombinant protein.

In the studies herein, early anti-TPP1 responses were most robust in dogs that were not pretreated with MMF. Introduction of drug at the time of or before AAV delivery reduced, inhibited or dramatically delayed anti-TPP1 immune responses. In this same dog disease model, immune responses to recombinant human TPP1, infused into the lumbar subarachnoid space or lateral ventricle, also induced robust anti-TPP1 responses (Vuillemenot et al., 2015). Anaphylactic reactions were noted with bolus infusion that was better managed or did not develop with slower, continuous infusion protocols. To date, there have been no reports of anti-TPP1 responses impacting children being treated bi-weekly with recombinant protein. Whether this reflects differences between immune responses to recombinant protein in CSF between children and dogs deficient in TPP1 is unknown. Immune responses to neoantigens in children receiving enzyme therapy for life-threatening lysosomal storage diseases can be managed as reported in the treatment of patients with Pompe's disease (Messenger et al., 2012). The approach developed herein could be applied if NAbs limited efficacy in TPP1-deficient children following AAV.TPP1 delivery. Finally, while the dose applied here resulted in clinical efficacy, this may not be the minimal effective dose, and thus lower doses would likely result in less robust immune responses. As carrier dogs do not exhibit symptoms, expressing 50% of normal TPP1 levels continuously throughout life appears to be sufficient.

Although treated, dogs that responded well to the gene therapy eventually succumbed to disease. While the data showed enzyme reaching the periphery from the CSF (elevated levels in heart and spleen), the levels may be insufficient to rescue systemic manifestations that may appear as the dogs live longer as a result of improved neurological function. Nonetheless, the approach disclosed herein holds promise from several perspectives over current a gene therapy trial for children with TPP1 deficiency and ERT. One advantage is that this approach would not require extensive time under sedation (device implant) for vector infusion, or intensive care for multiple gene therapy infusions. Finally, this approach could reduce risk to patients as it would obviate the need for life long enzyme infusions that would require chronic, repeated access to brain.

### References

1. Awano, T. et al., "A frame shift mutation in canine TPP1 (the ortholog of human CLN2) in a juvenile Dachshund with neuronal ceroid lipofuscinosis." Mol. Genet. Metab. (2006) 89(3):254-60.
2. Chang, M. et al., "Intraventricular enzyme replacement improves disease phenotypes in a mouse model of late infantile neuronal ceroid lipofuscinosis." Mol. Ther. (2008) 16(4):649-56.
3. Davidson, B.L. et al., "Recombinant adeno-associated virus type 2, 4, and 5 vectors: transduction of variant cell types and regions in the mammalian central nervous system." Proc. Natl. Acad. Sci. USA (2000) 97(7):3428-32.
4. Junaid, M.A. et al., "A novel assay for lysosomal pepstatin-insensitive proteinase and its application for the diagnosis of late-infantile neuronal ceroid lipofuscinosis." Clin. Chim. Acta. (1999) 281(1-2): 169-76.
5. Katz, M.L. et al., "Retinal pathology in a canine model of late infantile neuronal ceroid lipofuscinosis." Invest. Ophthalmol. Vis. Sci. (2008) 49(6):2686-95.
6. Katz, M.L. et al., "Enzyme replacement therapy attenuates disease progression in a canine model of late-infantile neuronal ceroid lipofuscinosis (CLN2 disease)." J. Neurosci. Res. (2014) 92(11):1591-8.
7. Kida, E.et al., "Distribution of tripeptidyl peptidase I in human tissues under normal and pathological conditions." J. Neuropthol. Exp. Neurol. (2001) 60(3):280-92.
8. Liu, G. et al., "Functional correction of CNS phenotypes in a lysosomal storage disease model using adeno-associated virus type 4 vectors." J. Neurosci. (2005) 25(41):9321-7.
9. Lorenz et al., 2011
10. Messenger, Y.H. et al., "Successful immune tolerance induction to enzyme replacement therapy in CRIM-negative infantile Pompe disease." Genet. Med. (2012) 14(1):135-42.
11. Micsenyi, M.C. et al., "Lysosomal membrane permeability stimulates protein aggregate formation in neurons of a lysosomal disease." J. Neurosci. (2013) 33(26):10815-27.
12. Ostergaard, J.R. et al., "Cardiac involvement in juvenile neuronal ceroid lipofuscinosis (Batten disease)." Neurology (2011) 76(14):1245-51.
13. Remington's Pharmaceutical Sciences, 15th Ed., Mack Publishing Co., New Jersey (1991).
14. Sambrook, et al. (1989) Molecular Cloning, a Laboratory Manual, Cold Spring Harbor Laboratories, New York).
15. Sanders, D.N. et al., "A reversal learning task detects cognitive deficits in a Dachshund model of late-infantile neuronal ceroid lipofuscinosis" Genes Brain Behav. (2011) 10(7):798-804.
16. Sleat, D.E. et al., "A mouse model of classical late-infantile neuronal ceroid lipofuscinosis based on targeted disruption of the CLN2 gene results in a loss of tripeptidyl-peptidase I activity and progressive neurodegeneration." J. Neurosci. (2004) 24(41):9117-26.
17. Tian, Y. et al., "Determination of the substrate specificity of tripeptidyl-peptidase I using combinatorial peptide libraries and development of improved fluorogenic substrates." J. Biol. Chem. (2006) 281(10):6559-72.
18. Vuillemenot, B.R. et al., "Nonclinical evaluation of CNS-administered TPP1 enzyme replacement in canine CLN2 neuronal ceroid lipofuscinosis." Mol. Genet. Metab. (2015) 114(2):281-93.
19. Worgall, S. et al., "Treatment of late infantile neuronal ceroid lipofuscinosis by CNS administration of a serotype 2 adeno-associated virus expressing CLN2 cDNA." Hum. Gene Ther. (2008) 19(5):463-74.
20. Wright, J.F., "Manufacturing and characterizing AAV-based vectors for use in clinical studies." Gene therapy (2008) 15(11):840-8.
21. Wright, J.F., "Transient transfection methods for clinical adeno-associated viral vector production." Human gene therapy (2009) 20(7):698-706.

## Claims

1. A plurality of AAV particles, said AAV particles (i) comprising an AAV capsid protein and a nucleic acid inserted between a pair of AAV inverted terminal repeats (ITRs), said nucleic acid encoding a polypeptide, wherein said polypeptide comprises a tripeptidylpeptidase 1 (TPP1) polypeptide having at least 90% identity to an amino acid sequence set forth in SEQ ID NO:5 or a pro-enzyme thereof, and (ii) being capable of transducing cells of said mammal and providing expression of said polypeptide; and a first immunosuppressive agent and a second immunosuppressive agent, for use in treating a lysosomal storage disease (LSD) in a mammal,
wherein the plurality of AAV particles is to be administered to the brain or spine of said mammal,
wherein said first immunosuppressive agent and said second immunosuppressive agent are to be administered to said mammal, wherein said first immunosuppressive agent is cyclosporine and said second immunosuppressive agent is mycophenolate mofetil (MMF),
wherein at least one of said first immunosuppressive agent and said second immunosuppressive agent are to be administered to said mammal prior to administration of said AAV particles, and
wherein neutralizing antibody titer in said mammal is reduced by administration of said first immunosuppressive agent and said second immunosuppressive agent.

2. The plurality of AAV particles, the first immunosuppressive agent and the second immunosuppressive agent for use according to claim 1, wherein said first immunosuppressive agent is to be administered prior to administration of said AAV particles and said second immunosuppressive agent is to be administered prior to, concurrently with, or after administration of said AAV particles.

3. The plurality of AAV particles, the first immunosuppressive agent and the second immunosuppressive agent for use according to claim 1 or 2, wherein (i) said first immunosuppressive agent is to be administered at least about two weeks prior to administration of said AAV particles and (ii) said second immunosuppressive agent is to be administered about two weeks before or within 40 days after administration of said AAV particles.

4. The plurality of AAV particles, the first immunosuppressive agent and the second immunosuppressive agent for use according to any one of claims 1-3, wherein administration of said AAV particles comprises administration to the cisterna magna, intraventricular space, brain ventricle, subarachnoid space, intrathecal space, ependyma, or cerebral spinal fluid (CSF) of said mammal, or wherein administration of said AAV particles comprises contacting ependymal cells, a pial cell, endothelial cell, or meningeal cell of said mammal with said AAV particles.

5. The plurality of AAV particles, the first immunosuppressive agent and the second immunosuppressive agent for use according to any one of claims 1-4, wherein said mammal is a non-rodent mammal, wherein said non-rodent mammal preferably is a primate, horse, sheep, goat, pig, dog, or a human, wherein said human preferably is a child, wherein said child preferably is from about 1 to about 4 years of age.

6. The plurality of AAV particles, the first immunosuppressive agent and the second immunosuppressive agent for use according to any one of claims 1-5, wherein said LSD is infantile or late infantile ceroid lipofuscinoses (LINCL), neuronopathic Gaucher, Juvenile Batten, Fabry, MLD, Sanfilippo A, Hunter, Krabbe, Morquio, Pompe, Niemann-Pick C, Tay-Sachs, Hurler (MPS-I H), Sanfilippo B, Maroteaux-Lamy, Niemann-Pick A, Cystinosis, Hurler-Scheie (MPS-1 H/S), Sly Syndrome (MPS VII), Scheie (MPS-I S), Infantile Batten, GM1 Gangliosidosis, Mucolipidosis type II/III, or Sandhoff disease.

7. The plurality of AAV particles, the first immunosuppressive agent and the second immunosuppressive agent for use according to any one of claims 1-6, wherein administration of said AAV particles comprises injection of said AAV particles into a tissue or fluid of the brain or spinal cord of said mammal.

8. The plurality of AAV particles, the first immunosuppressive agent and the second immunosuppressive agent for use according to any one of claims 1-7, wherein said cyclosporine is to be administered at a dosage of about 5-20 mg/kg twice a day for a period of at least 3 months, or wherein the dose of said cyclosporine administered is to be reduced after 1-2 months after administration of said AAV particles.

9. The plurality of AAV particles, the first immunosuppressive agent and the second immunosuppressive agent for use according to any one of claims 1-8, wherein said MMF is to be administered at a dosage of about 5-20 mg/kg a day.

10. The plurality of AAV particles, the first immunosuppressive agent and the second immunosuppressive agent for use according to any one of claims 1-9, wherein said AAV particles are to be administered at a dose of about 1×10⁸ to about 1×10¹⁵ vg/kg.

11. The plurality of AAV particles, the first immunosuppressive agent and the second immunosuppressive agent for use according to any one of claims 1-10, wherein said AAV particles are selected from the group consisting of AAV1, AAV2, AAV3, AAV4, AAV5, AAV6, AAV7, AAV8, AAV9, AAV10, AAV11, AAV12, AAV-rh74, AAV-rh10 and AAV-2i8 particles, or wherein one or more of said ITRs is selected from the group consisting of an AAV1, AAV2, AAV3, AAV4, AAV5, AAV6, AAV7, AAV8, AAV9, AAV10, AAV11, AAV12, AAV-rh74, AAV-rh10 and AAV-2i8 ITR.

12. The plurality of AAV particles, the first immunosuppressive agent and the second immunosuppressive agent for use according to any one of claims 1-11, wherein the nucleic acid inserted between a pair of AAV ITRs further comprises an expression control element driving expression of the nucleic acid encoding TPP1, wherein the expression control element comprises a sequence having 80% or more identity to SEQ ID NO:9.

## Patentansprüche

1. Vielzahl von AAV-Partikeln, die AAV-Partikel (i) umfassen ein AAV-Kapsidprotein und eine Nukleinsäure, die zwischen einem Paar von AAV-invertierten terminalen Wiederholungen (ITRs) inseriert ist, die Nukleinsäure kodiert ein Polypeptid, wobei das Polypeptid ein Tripeptidylpeptidase 1 (TPP1)-Polypeptid, das mindestens 90 % Identität zu einer in SEQ ID NO: 5 dargestellten Aminosäuresequenz oder einem Pro-Enzym davon aufweist, umfasst, und (ii) in der Lage ist, Zellen des Säugetiers zu transduzieren und die Expression des Polypeptids bereitzustellen; und ein erstes immunsupprimierendes Mittel und ein zweites immunsupprimierendes Mittel zur Verwendung bei der Behandlung einer lysosomalen Speicherkrankheit (LSD) in einem Säugetier,
wobei die Vielzahl von AAV-Partikeln in das Gehirn oder die Wirbelsäule des Säugetiers verabreicht wird,
wobei das erste immunsupprimierende Mittel und das zweite immunsupprimierende Mittel dem Säugetier verabreicht werden, wobei das erste immunsupprimierende Mittel Cyclosporin ist und das zweite immunsupprimierende Mittel Mycophenolat-Mofetil (MMF) ist,
wobei mindestens eines des ersten immunsupprimierenden Mittels und des zweiten immunsupprimierenden Mittels dem Säugetier vor der Verabreichung der AAV-Partikel verabreicht wird, und
wobei der Titer der neutralisierenden Antikörper in dem Säugetier durch die Verabreichung des ersten immunsupprimierenden Mittels und des zweiten immunsupprimierenden Mittels reduziert wird.

2. Vielzahl von AAV-Partikeln, das erste immunsupprimierende Mittel und das zweite immunsupprimierende Mittel zur Verwendung gemäß Anspruch 1, wobei das erste immunsupprimierende Mittel vor der Verabreichung der AAV-Partikel und das zweite immunsupprimierende Mittel vor, gleichzeitig oder nach der Verabreichung der AAV-Partikel verabreicht wird.

3. Vielzahl von AAV-Partikeln, das erste immunsupprimierende Mittel und das zweite immunsupprimierende Mittel zur Verwendung gemäß Anspruch 1 oder 2, wobei (i) das erste immunsupprimierende Mittel mindestens etwa zwei Wochen vor der Verabreichung der AAV-Partikel verabreicht wird und (ii) das zweite immunsupprimierende Mittel etwa zwei Wochen vor oder innerhalb von 40 Tagen nach der Verabreichung der AAV-Partikel verabreicht wird.

4. Vielzahl von AAV-Partikeln, das erste immunsupprimierende Mittel und das zweite immunsupprimierende Mittel zur Verwendung nach einem der Ansprüche 1-3, wobei die Verabreichung der AAV-Partikel die Verabreichung an die Cisterna Magna, den intraventrikulären Raum, den Hirnventrikel, den Subarachnoidalraum, den Intrathekalraum, das Ependym oder die cerebrale Rückenmarksflüssigkeit (CSF) des Säugetiers umfasst, oder wobei die Verabreichung der AAV-Partikel das Inkontaktbringen von ependymalen Zellen, einer Pialzelle, Endothelzelle oder Meningalzelle des Säugetiers mit den AAV-Partikeln umfasst.

5. Vielzahl von AAV-Partikeln, das erste immunsupprimierende Mittel und das zweite immunsupprimierende Mittel zur Verwendung nach einem der Ansprüche 1-4, wobei das Säugetier ein Nicht-Nagetier-Säugetier ist, wobei das Nicht-Nagetier-Säugetier vorzugsweise ein Primat, Pferd, Schaf, Ziege, Schwein, Hund oder ein Mensch ist, wobei der Mensch vorzugsweise ein Kind ist, wobei das Kind vorzugsweise etwa 1 bis etwa 4 Jahre alt ist.

6. Vielzahl von AAV-Partikeln, das erste immunsupprimierende Mittel und das zweite immunsupprimierende Mittel zur Verwendung nach einem der Ansprüche 1-5, wobei die LSD infantile oder spätinfantile Ceroid-Lipofuszinosen (LINCL), neuropathischer Gaucher, Juvenile Batten, Fabry, MLD, Sanfilippo A, Hunter, Krabbe, Morquio, Pompe, Niemann-Pick C, Tay-Sachs, Hurler (MPS-I H), Sanfilippo B, Maroteaux-Lamy, Niemann-Pick A, Cystinose, Hurler-Scheie (MPS-I H/S), Sly-Syndrom (MPS VII), Scheie (MPS-I S), Infantile Batten, GM1-Gangliosidose, Mukolipidose Typ II/III oder Sandhoff-Krankheit ist.

7. Vielzahl von AAV-Partikeln, das erste immunsupprimierende Mittel und das zweite immunsupprimierende Mittel zur Verwendung nach einem der Ansprüche 1-6, wobei die Verabreichung der AAV-Partikel die Injektion der AAV-Partikel in ein Gewebe oder eine Flüssigkeit des Gehirns oder Rückenmarks des Säugetiers umfasst.

8. Vielzahl von AAV-Partikeln, das erste immunsupprimierende Mittel und das zweite immunsupprimierende Mittel zur Verwendung nach einem der Ansprüche 1-7, wobei das Cyclosporin in einer Dosierung von etwa 5-20 mg/kg zweimal täglich über einen Zeitraum von mindestens 3 Monaten verabreicht wird, oder wobei die Dosis des verabreichten Cyclosporins nach 1-2 Monaten nach Verabreichung der AAV-Partikel reduziert wird.

9. Vielzahl von AAV-Partikeln, das erste immunsupprimierende Mittel und das zweite immunsupprimierende Mittel zur Verwendung nach einem der Ansprüche 1-8, wobei das MMF in einer Dosierung von etwa 5-20 mg/kg pro Tag verabreicht wird.

10. Vielzahl von AAV-Partikeln, das erste immunsupprimierende Mittel und das zweite immunsupprimierende Mittel zur Verwendung nach einem der Ansprüche 1-9, wobei die AAV-Partikel in einer Dosis von etwa 1×10⁸ bis etwa 1×10¹⁵ vg/kg verabreicht wird.

11. Vielzahl von AAV-Partikeln, das erste immunsupprimierende Mittel und das zweite immunsupprimierende Mittel zur Verwendung nach einem der Ansprüche 1-10, wobei die AAV-Partikel aus der Gruppe ausgewählt sind, die aus AAV1, AAV2, AAV3, AAV4, AAV5, AAV6, AAV7, AAV8, AAV9, AAV10, AAV11, AAV12, AAV-rh74, AAV-rh10 und AAV-2i8 Partikeln besteht, oder wobei eines oder mehrere der ITRs aus der Gruppe ausgewählt sind, die aus einem AAV1, AAV2, AAV3, AAV4, AAV5, AAV6, AAV7, AAV8, AAV9, AAV10, AAV11, AAV12, AAV-rh74, AAV-rh10 und AAV-2i8 ITR besteht.

12. Vielzahl von AAV-Partikeln, das erste immunsupprimierende Mittel und das zweite immunsupprimierende Mittel zur Verwendung nach einem der Ansprüche 1-11, wobei die zwischen einem Paar von AAV-ITRs eingefügte Nukleinsäure ferner ein Expressionskontroll-Element umfasst, das die Expression der TPP1 kodierenden Nukleinsäure steuert, wobei das Expressionskontroll-Element eine Sequenz mit 80 % oder mehr Identität zu SEQ ID NO:9 umfasst.

## Revendications

1. Pluralité de particules d'AAV, lesdites particules d'AAV (i) comprenant une protéine de capside d'AAV et un acide nucléique inséré entre une paire de répétitions terminales inversées (ITR) d'AAV, ledit acide nucléique codant pour un polypeptide, ledit polypeptide comprenant un polypeptide tripeptidyl peptidase 1 (TPP1) ayant au moins 90% d'identité avec une séquence d'acides aminés présentée en SEQ ID NO:5 ou une proenzyme de celui-ci, et (ii) étant capable de transduire les cellules dudit mammifère et fournir l'expression dudit polypeptide; et un premier agent immunosuppresseur et un deuxième agent immunosuppresseur, pour utilisation dans le traitement d'une maladie de surcharge lysosomale (LSD) chez un mammifère,
la pluralité de particules d'AAV devant être administrée au cerveau ou à la colonne vertébrale dudit mammifère,
ledit premier agent immunosuppresseur et ledit deuxième agent immunosuppresseur devant être administrés audit mammifère, ledit premier agent immunosuppresseur étant la cyclosporine et ledit deuxième agent immunosuppresseur étant le mycophénolate mofétil (MMF),
au moins l'un desdits premier agent immunosuppresseur et deuxième agent immunosuppresseur devant être administré audit mammifère avant l'administration desdites particules d'AAV, et
le titre des anticorps neutralisants chez ledit mammifère étant réduit par l'administration dudit premier agent immunosuppresseur et dudit deuxième agent immunosuppresseur.

2. Pluralité de particules d'AAV, premier agent immunosuppresseur et deuxième agent immunosuppresseur pour utilisation selon la revendication 1, ledit premier agent immunosuppresseur devant être administré avant l'administration desdites particules d'AAV et ledit deuxième agent immunosuppresseur devant être administré avant, simultanément à ou après l'administration desdites particules d'AAV.

3. Pluralité de particules d'AAV, premier agent immunosuppresseur et deuxième agent immunosuppresseur pour utilisation selon la revendication 1 ou 2, (i) ledit premier agent immunosuppresseur devant être administré au moins environ deux semaines avant l'administration desdites particules d'AAV et (ii) ledit deuxième agent immunosuppresseur devant être administré environ deux semaines avant ou dans les 40 jours après l'administration desdites particules d'AAV.

4. Pluralité de particules d'AAV, premier agent immunosuppresseur et deuxième agent immunosuppresseur pour utilisation selon l'une quelconque des revendications 1 à 3, dans lesquels l'administration desdites particules d'AAV comprend l'administration dans la cistema magna, l'espace intraventriculaire, le ventricule cérébral, l'espace sous-arachnoïdien, l'espace intrathécal, l'épendyme ou le liquide céphalorachidien (LCR) dudit mammifère, ou dans lesquels l'administration desdites particules d'AAV comprend la mise en contact de cellules épendymaires, d'une cellule piale, d'une cellule endothéliale ou d'une cellule méningée dudit mammifère avec lesdites particules d'AAV.

5. Pluralité de particules d'AAV, premier agent immunosuppresseur et deuxième agent immunosuppresseur pour utilisation selon l'une quelconque des revendications 1 à 4, dans lesquels ledit mammifère est un mammifère non-rongeur, ledit mammifère non-rongeur étant de préférence un primate, un cheval, un mouton, une chèvre, un cochon, un chien ou un humain, ledit humain étant de préférence un enfant, ledit enfant étant de préférence âgé d'environ 1 à environ 4 ans.

6. Pluralité de particules d'AAV, premier agent immunosuppresseur et deuxième agent immunosuppresseur pour utilisation selon l'une quelconque des revendications 1 à 5, dans lesquels ladite LSD sont des lipofuscinoses céroïdes infantiles ou infantiles tardives (LINCL), la maladie neuronopathique de Gaucher, de Batten juvénile, de Fabry, la MLD, de Sanfilippo A, de Hunter, de Krabbe, de Morquio, de Pompe, de Niemann-Pick C, de Tay-Sachs, de Hurler (MPS-I H), de Sanfilippo B, de Maroteaux-Lamy, de Niemann-Pick A, Cystinose, de Hurler-Scheie (MPS -I H/S), le syndrome de Sly (MPS VII), la maladie de Scheie (MPS-I S), de Batten infantile, la Gangliosidose à GM1, la Mucolipidose de type II/III ou la maladie de Sandhoff.

7. Pluralité de particules d'AAV, premier agent immunosuppresseur et deuxième agent immunosuppresseur pour utilisation selon l'une quelconque des revendications 1 à 6, dans lesquels l'administration desdites particules d'AAV comprend l'injection desdites particules d'AAV dans un tissu ou un liquide du cerveau ou de la moelle épinière dudit mammifère.

8. Pluralité de particules d'AAV, premier agent immunosuppresseur et deuxième agent immunosuppresseur pour utilisation selon l'une quelconque des revendications 1 à 7, dans lesquels ladite cyclosporine doit être administrée à un dosage d'environ 5 à 20 mg/kg deux fois par jour pour une période d'au moins 3 mois, ou dans lesquels la dose de ladite cyclosporine administrée doit être réduite après 1 à 2 mois après l'administration desdites particules d'AAV.

9. Pluralité de particules d'AAV, premier agent immunosuppresseur et deuxième agent immunosuppresseur pour utilisation selon l'une quelconque des revendications 1 à 8, dans lesquels ledit MMF doit être administré à un dosage d'environ 5 à 20 mg/kg par jour.

10. Pluralité de particules d'AAV, premier agent immunosuppresseur et deuxième agent immunosuppresseur pour utilisation selon l'une quelconque des revendications 1 à 9, lesdites particules d'AAV devant être administrées à une dose d'environ 1 × 10⁸ à environ 1 × 10¹⁵ vg/kg.

11. Pluralité de particules d'AAV, premier agent immunosuppresseur et deuxième agent immunosuppresseur pour utilisation selon l'une quelconque des revendications 1 à 10, dans lesquels lesdites particules d'AAV sont sélectionnées du groupe constitué par des particules AAV1, AAV2, AAV3, AAV4, AAV5, AAV6, AAV7, AAV8, AAV9, AAV10, AAV11, AAV12, AAV-rh74, AA V-rh 1 0 et AAV-2i8, ou dans lesquels l'une ou plusieurs desdites ITR sont sélectionnées du groupe constitué par des ITR d'AAV1, AAV2, AAV3, AAV4. , AAV5, AAV6, AAV7, AAV8, AAV9, AAV10, AAV11, AAV12, AAV-rh74, AAV-rh10 et AAV-2i8.

12. Pluralité de particules d'AAV, premier agent immunosuppresseur et deuxième agent immunosuppresseur pour utilisation selon l'une quelconque des revendications 1 à 11, dans lesquels l'acide nucléique inséré entre une paire d'ITR d'AAV comprend en outre un élément de contrôle d'expression pilotant l'expression de l'acide nucléique codant pour la TPP1, l'élément de contrôle d'expression comprenant une séquence ayant 80% ou plus d'identité avec SEQ ID NO:9.
